Europäisches Patentamt

European Patent Office

Office européen des brevets

(11) Publication number:

# 0 031 807
A2

(12) **EUROPEAN PATENT APPLICATION**

(21) Application number: 81100230.2

(22) Date of filing: 05.03.79

(51) Int. Cl.³: **A 24 C 1/04,** G 06 F 15/20, B 26 D 5/34

(30) Priority: 09.03.78 US 884849

(43) Date of publication of application: 08.07.81 Bulletin 81/27

(84) Designated Contracting States: **BE CH DE FR GB IT LU NL SE**

(60) Publication number of the earlier application in accordance with Art. 76 EPC: **0004170**

(71) Applicant: **GULF & WESTERN CORPORATION, 1 Gulf & Western Plaza, New York, N.Y. 10023 (US)**

(72) Inventor: **Sinclair, Rober I., Roxciticus Road R.D. No.2, Mendham New Jersey 07945 (US)**
Inventor: **Anderson, Verner, R.D. No. 3-3103, Berwick Pennsylvania 18603 (US)**
Inventor: **Logan, David J., 125 Karen Lee Road, Glastonbury Connecticut 06133 (US)**
Inventor: **Rich, Leonard G., 7 Rye Ridge Parkway, West Hartford Connecticut 06117 (US)**

(74) Representative: **Abble, Andrew Kenneth, A.A. THORNTON & CO. Northumberland House 303/306 High Holborn, London, WC1V 7LE (GB)**

(54) **Apparatus for detecting the general outline and abrupt surface variations of a flaccid sheet-like workpiece formed from at least a portion of a natural tobacco leaf.**

㊗ Such apparatus provides a representation of the general outlines and abrupt surface variations of a tobacco leaf (L) supported on a support surface wich representation is used for locating cut locations on the leaf from which wrappers (W) can be cut. The apparatus includes a foraminate, light ray conducting leaf receiving conveyor (300) on which the leaf is held in a spread condition by vacuum. A light source (360) on one side of the conveyor passes light through the conveyor and the leaf thereon such that the transmitted light intensity at the other side of the conveyor which is measured by a scanning head (340) comprising a series of units extending across the conveyor in an X direction is indicative of the shielding effect of the conveyor and leaf. The conveyor is shifted in a Y direction orthogonal to the X direction past the units and digital signals indicative of the shielded light intensity at each of the units (340) at each shifted position, or further digital signals derived from these signals, are recorded in a memory and a representation of the general outline and abrupt surface variations of the leaf is created in a computer.

- 1 -

## Apparatus for detecting the general outline and abrupt surface variations of a flaccid sheet-like workpiece formed from at least a portion of a natural tobacco leaf

Attention is directed to the claims of copending Application No. 79300323.7 from which this application is divided and also to the claims of Applications Nos.          ,                    and which are also divided therefrom.

The present invention is directed to apparatus for detecting the general outline and abrupt surface variations of a flaccid sheet-like workpiece formed from at least a portion of a natural tobacco leaf and for providing a representation of the general outline and abrupt surface variations.

An embodiment of the invention will be described in connection with the cutting of cigar wrappers from tobacco leaves.

In the production of cigars, a core is usually provided. In practice, the core is formed either of small tobacco pieces or whole tobacco leaves bunched in a longitudinal direction. These cores are usually wrapped with a binder and then spirally wrapped by an elongated sheet, known as a "wrapper". The cigar wrapper is formed by cutting a given profile from a tobacco sheet product. In some instances, a wrapper is cut from a synthetic tobacco sheet. However, it is desirable to provide a natural tobacco leaf as the outer cigar wrapper to present an appealing appearance which facilitates marketing of the finished cigar. It is essential for a quality cigar that a natural tobacco leaf be used for the outer wrapper and that the finished cigar have no noticeable defect, such as a hole, a color variation, an edge portion of the leaf, or a stem. Indeed, certain unduly noticeable heavy veins should not be incorporated into a wrapper for a quality cigar. All of these concepts in wrapper selection are essential in the marketing of a cigar in the very competitive cigar industry. The wrapper is important in determining the overall visual concept that a purchaser forms regarding the quality of the cigar. Consequently, the wrapper must have an appearance that imparts an impression of quality to the resulting cigar.

As is well known in the cigar industry, the wrapper, which is helically disposed in overlapping fashion around the cigar, must also have a smooth outer appearance which is somewhat difficult to obtain since the shape of the cigar often varies along its length. For that reason, the wrapper must
be cut in a complex shape and must be accurately spiraled
around the cigar core and/or binder to produce the desired
smooth outer appearance. This requirement, combined with
the demand for a cigar wrapper with no surface defects, has
made one of the more critical aspects of cigar making the
system by which the cigar wrapper is cut from a natural tobacco leaf. This system is made quite complex by the fact
that each natural tobacco leaf has different surface variations which must be excluded from a wrapper.

Due to the extreme criticality in producing quality cigar
wrappers for use in cigar manufacturing, the wrappers are generally cut by a manual orienting procedure that has not
changed substantially over the years. The process requires a
skilled operator who manually orients a half of a leaf formed
by removing the center stem. This leaf portion or half is
examined for holes, coarse veins, or other visible imperfections on one surface. After this inspection, the leaf portion
is usually spread onto a cutting surface including a cutting
die surrounded by perforated surfaces through which vacuum
can be applied to the spread leaf. The leaf is manually
positioned over the cutting die to insure that the outline of
the die, which has the shape of the cigar wrapper, does not
include an edge portion or any other visible surface imperfection in the natural tobacco leaf. After this placement
has been made, the vacuum is applied to hold the leaf in place
on the cutting surface. A roller is forced over the leaf and
cuts out a leaf portion determined by the profile of the
cutter over which the leaf was positioned. After a first cut
has been made, the vacuum is released, the wrapper is removed
and the cut tobacco portion is again oriented by the operator for a second cut from the leaf half, if a second cut is possible without including any surface imperfection. The wrapper has an elongated shape and the leaf veins must have a predetermined diagonal orientation in the wrapper. Consequently, the general disposition of the wrapper cut must be generally parallel to the original stem of the natural tobacco leaf within a few degrees, such as $10^{\circ}-15^{\circ}$. In this manner, the veins, which are found on the leaf, will have the proper pattern when the wrapper is spirally wound around the core and/or binder to form a finished cigar. This process of manually orienting and then cutting is continued until no other wrapper can be cut from a leaf half. At this time, the leaf is removed from the cutting station for use in other tobacco products or discarded. Other procedures are used in the tobacco industry for producing the cigar wrappers. This particular description is representative in that each of the procedures involves manual manipulation of a leaf or a leaf half into a particular position wherein a cut is made in the natural tobacco leaf so as to avoid surface imperfections. In all instances, an operator, who must be skilled and highly trained, is required for the production of a quality wrapper produced from a natural tobacco leaf. In practice, the cost of producing the quality wrapper is a relatively high proportion of the cigar manufacturing costs in that the remainder of the cigar making process is generally mechanized and can be accomplished at relatively high processing speeds.

In view of this, there is a substantial demand for an arrangement wherein the cigar wrapper can be cut from a tobacco leaf in a high speed operation involving no manual manipulation without sacrificing the high quality required for the production of such wrappers. The advantage to the cigar manufacturing process of avoiding, or reducing, the manual manipulation required in producing the cigar wrappers is well known in the industry.

The provision of a representation of the general outline and surface variations of a leaf in accordance with the present invention enables the location of one or more cut locations on the leaf from which a wrapper can be cut to be made using the representation rather than the leaf itself and thus without requiring manual manipulation of the leaf.

The invention provides an apparatus for detecting the general outline and abrupt surface variations of a flaccid sheet-like workpiece formed from at least a portion of a natural tobacco leaf , said apparatus characterised in that it includes a foraminate, energy ray conducting workpiece receiving an element having a first workpiece supporting surface with an area and shape capable of receiving said workpiece in a spread condition and a coterminous second surface; means for creating a positive pressure differential from said first surface to said second surface in order to hold a workpiece against said first surface; an energy ray source means on one side of said element for passing energy rays through said workpiece receiving element and through said workpiece, such that the transmitted energy intensity at the other side of said element is indicative of the shielding effect of said receiving element and said workpiece; a series of energy intensity measuring units extending in a first direction across said receiving element and spaced from said first surface for measuring the transmitted energy intensity; means for creating a digital signal indicative of the shielded energy intensity at each of said units; means for shifting the relative position of said units and said leaf in a direction generally orthogonal to said first direction; means for recording said digital signals or further digital signals derived therefrom at each relative position of said units and said leaf; and means for creating a representation of said general outline and abrupt surface variations based upon said signals.

In order that the invention may be well understood, some embodiments thereof which are given by way of example only, will now be described with reference to the accompanying drawings, in which:

Figure 1 is a top plan view illustrating a natural tobacco leaf from which the wrappers are to be cut;

Figure 2 is a schematic view illustrating the shape of the cigar wrapper to be cut together with the encompassing profile or facsimile shape shown in dashed lines to be used in locating the cut position on the leaf shown in Figure 1;

Figure 3 is a top plan view of the leaf shown in Figure 1 with the cutter profiles located in cut positions;

Figure 4 is a block diagram showing schematically certain general features of a system for providing cigar wrappers cut from tobacco leaves;

Figure 5 is a block diagram outlining control concepts employed in the system;

Figure 6 is a flow chart block diagram illustrating interfacing concepts between the computer and the programmable controller as shown in Figure 5;

Figure 7 is a schematic, side elevational view showing the scanning mechanism and the transfer arrangement of the system;

Figure 7A is an enlarged cross-sectional view showing certain features of the structure illustrated in Figure 7;

Figure 7B is a schematic representation of the light intensity transducer units used in the scanning mechanism of Figure 7;

Figure 8 is a logic diagram illustrating in block diagram and logic diagram form the control system for the scanning operation to be employed in the structure as schematically illustrated in Figures 7, 7A and 7B;

Figure 9 is a schematic differential circuit which would create light intensity outputs as graphically illustrated in Figure 8;

Figure 10 is a chart showing an operating characteristic of the light intensity scanning arrangement employed in an embodiment of the present invention without the preferred modification as contemplated by the system as shown in Figure 11;

Figure 11 is a block diagram of the circuit for modifying the video output of the scanning unit to produce an output modified from that of Figures 8 and 9;

Figure 12 is a graph illustrating the operating characteristics of the circuit shown in Figure 11;

Figure 13 is a block diagram of the memory loading concept which could be employed in the system when raw data of all locations is used to creating a leaf facsimile;

Figure 14 is a logic diagram illustrating a system for creating transitions in the scanning operation for use as intermediate data in the preferred embodiment of the present system;

Figure 14A is a simplified logic diagram illustrating a transition data combining digital circuit employed in conjunction with Figure 14;

Figure 15 is a block diagram illustrating a data transfer system which could employ the data developed by the structure illustrated in Figure 14 and digital circuitry for processing and inputting such data;

Figures 16 and 17 are data transfer circuits similar to that shown in Figure 15 but employing the data developed by the schematic digital circuit of Figure 14A;

Figure 18 is a block diagram illustrating the direct access memory loading concept used in the preferred system wherein transition data is loaded into separate memory units or areas;

Figures 19 and 20 are tabulations of direct memory entered data employed in the preferred system of the present invention;

Figure 21 is a schematic view illustrating a natural tobacco leaf and certain scanning concepts employed in the preferred scanning concept of the system;

Figure 22 is a vectorized outline of the natural leaf shown in Figure 21;

Figures 23, 24 and 25 illustrate further concepts used in processing data employed in the preferred system; and

Figure 26 is an outline of the computer program steps for locating cut locations.

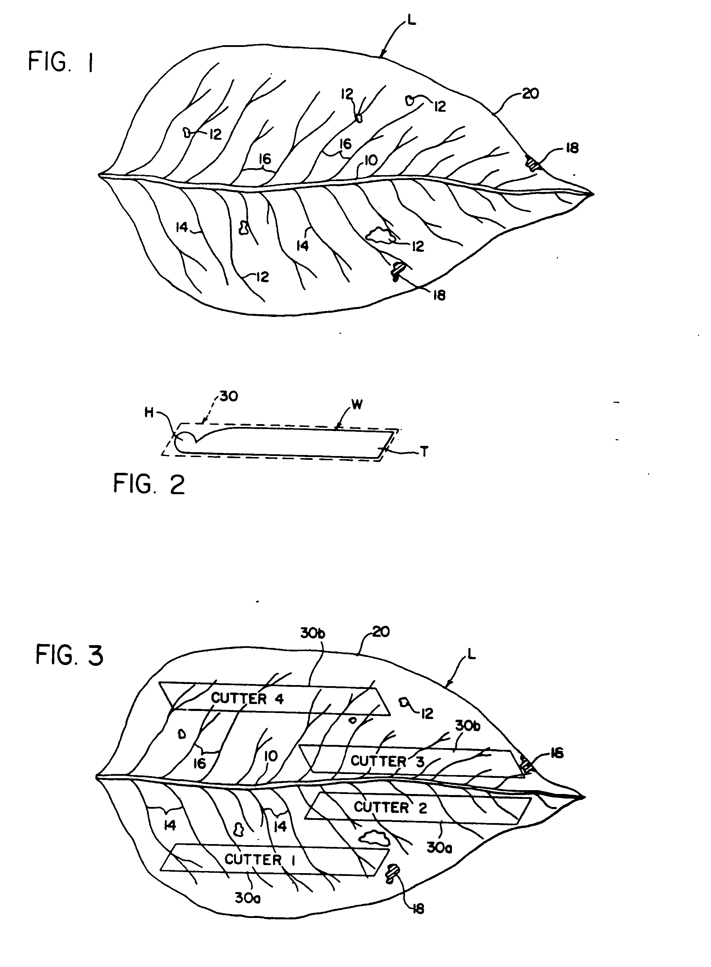

Referring to Figures 1 to 3, a natural tobacco leaf L has known physical characteristics in that it is flaccid, pliable and has internal colour variations, energy ray detectable variations and defects like holes, and dark or black areas. In addition, the natural tobacco leaf has a top surface which is to be used as the exposed portion of the wrapper to be cut from the leaf. Natural leaf L is illustrated as including a centre stem 10 which _____

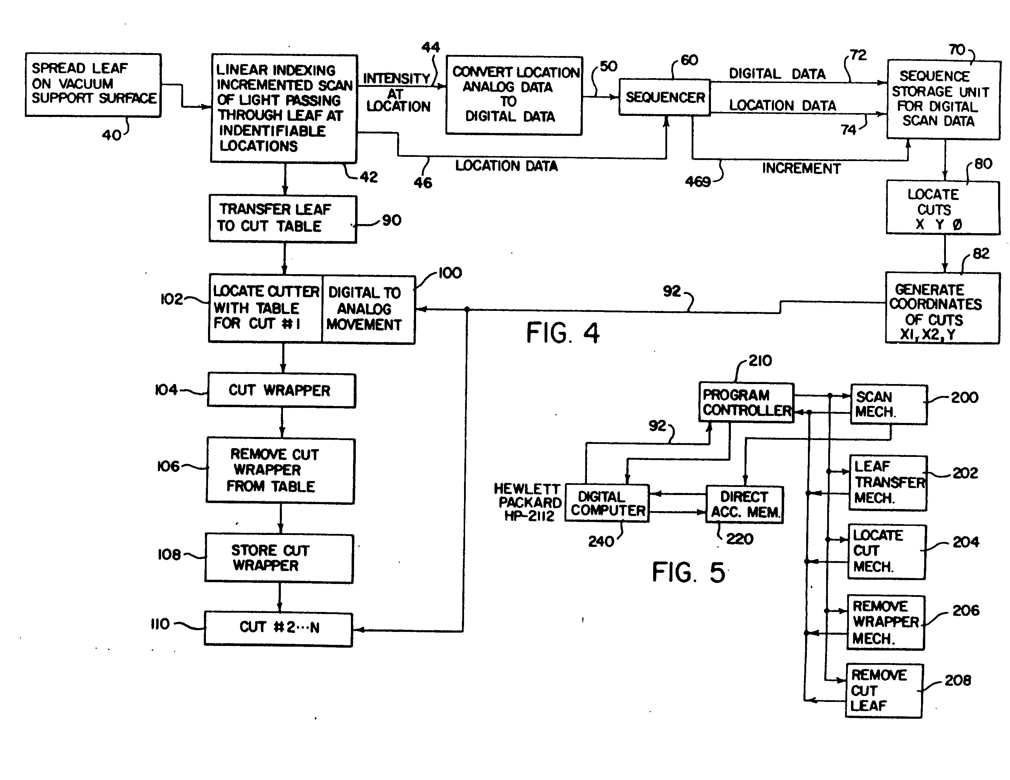

extends in a somewhat undulating path longitudinally through the leaf, a plurality of holes 12 of varying size, diagonally extending right and left veins 14, 16, respectively, dark areas 18 and an outline or edge 20. The dark areas 18 may be on either surface of the leaf and are visible from the top surface to a degree determined by their location. Indeed, the dark areas may be internal of the leaf and present only a slight color variation when viewed from the top surface of the leaf. Even in that situation, the dark areas could be considered unacceptable for a wrapper of a cigar. Also, abrupt surface color variations can occur as pronounced light areas in the leaf which may be detectable from one or both surfaces of the leaf and which may be undesirable for a cigar wrapper. From the leaf L cigar wrappers W, as shown in FIGURE 2, are to be cut by a cookie cutter type die or clicker die commonly used for cutting of flat sheets in other industries. Wrapper W has a precise shape which allows spiral winding of the wrapper around the binder and/or core of the cigar without producing wrinkles and surface defects. The wrapper generally includes a head H and a portion T called a "tuck". Tuck T is ultimately located in the end of the cigar to be lighted, whereas head H is twisted and wrapped into the portion of the cigar to be held in the smoker's mouth. These shapes are critical and it should be free of dark areas, any holes and heavy veins. The shape of wrapper W, which varies for different length and shaped cigars, can be circumscribed by a four-sided profile 30. The profile is different for left and right hand wrappers and are configurations 30a, 30b, as shown in FIGURE 3. The cutters are labeled CUTTERS 1-4. In this illustrated embodiment the cutters outlined by the shapes 30a, 30b represent cut positions of the same wrapper shapes. In some instances, two or more wrapper shapes could be cut from a single leaf.  For the purpose of describing
the present system, the cutter to be used in the wrapper has
the shape of wrapper W;  however, the profile 30 or profiles
30a, 30b are used to locate the cut positions for the wrappers
on the surface of leaf L in a manner to avoid any unwanted
defects in the resulting wrapper within the confines of
general profiles 30a, 30b.  In producing wrappers W from leaf
L the wrapper profile must be located on the leaf to avoid
defects such as holes, the stem, dark areas, color variations
of a given magnitude and other such contingencies to produce
a wrapper having the quality set forth in the introductory
portion of this application.  In most arrangements for cutting the wrapper W from a natural tobacco leaf, the stem is
removed and the leaf halves are booked or stacked according
to the right hand vein arrangement or the left hand vein
arrangement.  The present system does not contemplate the
stemming of the leaf;  however, it could be stemmed and a
half of a leaf could be processed in the present system.
Throughout the rest of the discussion of the present system,
wrapper profile 30 is used to locate the cut positions on
leaf L of the cutter for a wrapper W since the placement of
this profile at various non-conflicting locations will provide proper cut positions for the circumscribed actual wrapper
configuration.  In practice of the present system, the actual
profile 30 or profiles 30a, 30b are slightly greater in size
than the contour of the wrapper cutter so that the cut made
by the cutter itself will not be at the edge of the leaf or
closely adjacent a prohibitive defect to be excluded.  In
other words, the profile used in selecting the cut positions
in accordance with the system herein described is slightly
larger than the actual cutter profile used in making the
wrapper cut in leaf L so that the cut is offset at least a small amount from the outline of profile 30.

In accordance with the system contemplated by the present invention, a profile or image of the leaf including the outline, stem, surface variations and defects is created and recorded and stored. Thereafter, the profile 30 is manipulated within stored profile or image to locate positions in which the profile 30 avoids unwanted defects and remains in the confines of the leaf. These selected cut positions are then used to control a cutting machine that positions the leaf and wrapper cutter in the desired relative position for a cutting operation to remove the wrapper from the area bound by profile 30 in its final selected position or positions.

A general functional block diagram of the overall system is schematically illustrated in FIGURE 4. A description of this block diagram will provide general information regarding the basic concept of the system hereinafter described and can be used as an over view of the system of cutting wrappers W from desired positions on leaf L without requiring the previously used manual manipulation and without sacrificing quality of the resulting wrappers. In accordance with the functional block diagram, a natural leaf is spread onto a vacuum support surface which preferably is capable of transmitting light rays so that the rays pass simultaneously through the surface and through the leaf. These rays are read by a light intensity sensitive device which determines the light intensity at selected locations on the support surface. The total light intensity pattern provides a light intensity profile or image of the natural tobacco leaf supported in a spread condition on the surface. The concept of spreading the natural leaf onto a vacuum support surface for cutting is known and is represented by block 40. The concept of passing light through the leaf supported on a vacuum

surface ~~is novel and~~ is represented in block 42. As indicated by block 42, the leaf is indexed or moved incrementally in one direction (hereinafter called the Y direction) and the light intensities at selected areas of the leaf across another direction (hereinafter called the X direction) are recorded at identifiable locations identifiable by both X and Y positions on an imaginary grid on the vacuum surface. The intensity of the light passing through the leaf at these various identifiable locations which cover the total leaf as it has been moved are indicative of the contour of the leaf and color variation or defects including holes, dark areas, the stem, the veins and other surface color variations. By passing light through the leaf, variations on both surfaces are detected as are variations within the leaf which can cause discernible color variations that would be objectionable in a wrapper for a cigar. Block 42 is indicative of this scanning operation for obtaining light intensity at selected locations encompassing the total surface area of natural tobacco leaf L. The scanning operation creates a series of light intensity signals which are each analog voltages. One voltage signal corresponds to each scanned location and appears in line 44. Digital data indicative of the location of the scanned locations simultaneously appears in line 46. The analog light intensity signal in line 44 is converted to digital data indicating whether or not the scanned location has a light intensity value indicative of white, black or an intermediate color, termed "gray". Thus, each of the scanned locations on the leaf, which may be as small as desired and is known as a Pixel, will produce a light intensity, digital signal in line 50. The Pixel data will be white, black or gray. If desired, variations of gray could be obtained; however, such resolution is not required in the present system. The address data in line 46 identifies the location of the Pixel being detected and transmitted in line 50. As can be seen, if the leaf is segregated into a large number of identifiable locations, or Pixels, which have known addresses (in the X and Y directions) and known light intensity conditions, such as white, black or gray, a profile of the leaf itself can be constructed remote from the leaf. For illustrative purposes, a sequencer 60 is indicated as transmitting the digital information regarding the color of the Pixel and the location of the Pixel to any type of storage unit 70 by lines 72, 74. The particular storage arrangement will then contain a matrix indicative of the profile of the leaf including an outline and defects. This data matrix storage unit 70 is indicated to be a sequenced unit incremented by line 469 for each Pixel in the X direction. In practice, storage unit 70 is a direct accessed memory usable by a digital computer in accordance with known computer practice. The indexing by each X Pixel will cover all Y indexes in sequence. Proceeding further into the schematic representation of the general system as shown in FIGURE 4, cut locations or positions are determined as represented by block 80 using the stored digital data in storage unit 70. This can be done in a variety of computer arrangements which will be described in more detail later. Basically, a digital representation of the cutter profile 30 is compared to the stored digital profile of leaf L in storage unit 70 and these two profiles are shifted until the digitized profile 30 does not include a defect, such as a hole, edge of the leaf, or undue color variations. After one cut position is located, a next profile 30 is also shifted with respect to the digitized leaf profile or image in storage unit 70 to locate still a further cut position which avoids the defects set forth above. This process is continued until the digitized representation of profile 30 is set to the extent possible to obtain the desired number of cuts in leaf L. The various cut locations so located by shifting the digitized profiles 30 within the digitized leaf profile of storage unit 70 are in the form of X, Y and Ø corresponding to the X, Y location of a selected point on a line of profile 30 and the angle this line makes with the scanned Y direction. The X, Y and Ø coordinates are then converted to usable cut coordinates represented as X1, X2 and Y by a coordinate arithmetic conversion. This conversion is determined by the usable data necessary to obtain the same X, Y and Ø location on the cutting machine when a linear converting mechanism is used for purposes to be explained later. Arithmetic conversion to cut coordinates usable in the specific machinery contemplated for actually making the cut in the leaf is represented by function block 82. After the scanning operation schematically illustrated as block 42 has been completed, leaf L is transferred to a cutting platen or table. This process is represented by functional block 90. After the leaf has been transferred to the cut platen, the arithmetically generated coordinates from generator 82 are directed through an interface indicated generally as line 92 to a digital-to-analog movement mechanism 100. This mechanism shifts the cut table carrying the leaf to a known cut position determined by coordinates from interface 92. As will be explained later, the movement by mechanism 100 is translated along three axes corresponding to X1, X2 and Y coordinates. The moving to a cut position is represented by block 102. The transfer of leaf L to the cut table is in an oriented position and the cut table is a vacuum surface which holds leaf L in this oriented position. The coordinates from generator 82 operate on the table as they would on the scanning surface. After the leaf and table have been shifted to the first cut position, the wrapper cutter is shifted downwardly to cut a cigar wrapper from the leaf in this
first cut position.    This action is represented by
functional block 104.    The cutter is then raised to
remove the wrapper from the leaf, which leaf is still
held on the table by vacuum.    The wrapper is held by
a vacuum means as the cutter moves upwardly.    This
removes the wrapper from the cutting table as represented
by functional block 106.    Thereafter, the wrapper is
removed from the cutter by a vacuum transfer arrangement
in accordance with known wrapper handling procedures.
This functional step is illustrated as block 108.
Interface 92 then causes a recycling of the cutter table
to the second cut position and a second wrapper is cut,
removed and stored for subsequent use.    This procedure
is continued until all the cuts lcoated within leaf L
have been made.    Thereafter, the next leaf is processed.

In accordance with the preferred embodiment,
while the cutting operation is taking place on one leaf,
a second leaf is being spread on the vacuum support
surface and scanned and the cut positions are being
located and the coordinates X1, X2 and Y are being
created.    Thus, a tandem arrangement is contemplated
wherein the scanning is taking place on one leaf while
the cutting operation is taking place on a previous leaf.
To facilitate this dual operation, a general control
system, as shown schematically in Figure 5, is employed
in the preferred embodiment.    The system includes, as
basic machine elements, a scanning mechanism 200, a
leaf transfer mechanism 202, a locate and cut mechanism
204, a wrapper removing mechanism 206 and a mechanism
208 for removing the spent leaf after all the cuts have
been made from the leaf.    All of these mechanisms, which
will be explained in more detail later, are controlled
in accordance with a standard practice by a commercially available programmable controller 210, such as one using an Intel 8080 microprocessor. The sequence of mechanical operations are conducted in accordance with the program of the programmable controller in accordance with standard practice in machine control operation. The scanning mechanism 200 provides information to a direct access memory 220 which has a two-way communication with a standard digital computer 240 that locates the cut positions, converts them to X1, X2 and Y coordinates and provides the coordinates to the controller 210. A two-way communication with the programmable controller is provided to load the cut positions into the programmable controller when the programmable controller is ready to accept the cut coordinates for processing a scanned leaf L. The control and function of information provided in FIGURES 4 and 5 is illustrative in nature to show the general process being performed in accordance with the novel system and a general control arrangement which allows the various mechanisms to be operated to cut wrappers from leaf L. This use of a controller 210 reduces computer time and software and provides a standard type of machine control for the various mechanisms used to perform the sequence of events set forth generally in FIGURE 4.

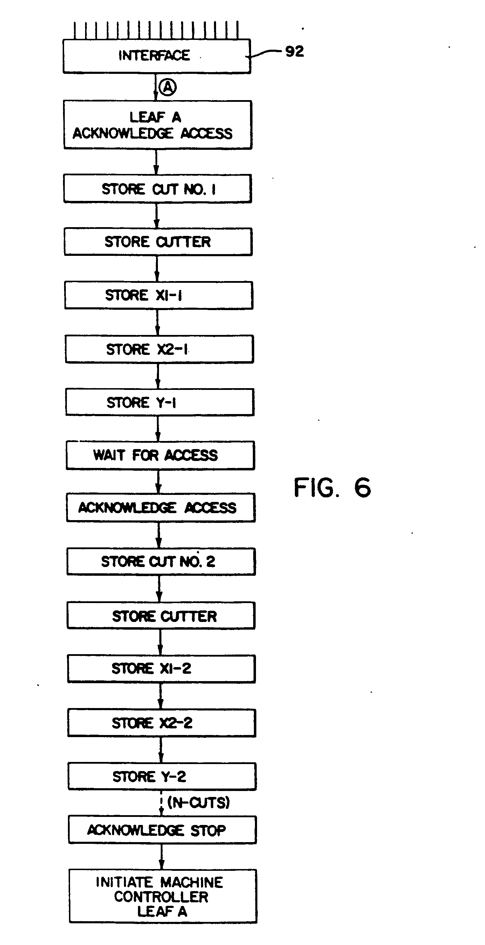

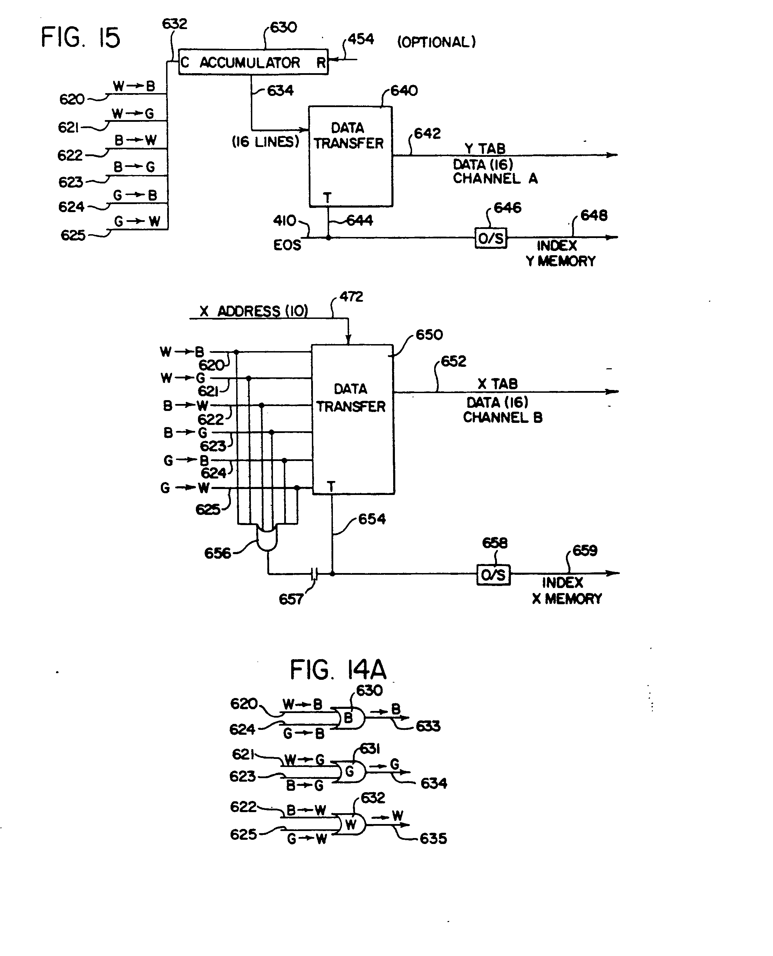

Referring now to FIGURE 6, this figure illustrates, somewhat generally, the interface 92 between the programmable controller 210 and the digital computer 240 as represented in FIGURE 5. Also, the information from computer 240 is illustrated in FIGURE 13. As a first step, the programmable controller 210 acknowledges that it desires access to the digital computer 240 for data regarding the cut coordinates for leaf designated as "leaf A". Thereafter, the programmable controller obtains and stores the number of the cut (1, 2 ... n), the particular cutter (cutter 1, 2, 3, etc), the coordinates X1, X2 and Y for the first cut. It is possible to use different cutters to obtain different wrapper sizes. For that reason, the particular cutter to be used is inputted to the
programmable controller.  In addition, as noted in FIGURE 3,
there are left and right hand wrapper cutters to accommodate
the different vein configurations.  Thus, if a cut is to be
made from one side of the leaf, one cutter is selected, whereas another cutter is selected for a wrapper from the other
side of the leaf.  Because of this, the programmable controller
receives information regarding which cutter is to be used,
which cuts are to be made and where the cut is to be made on
the leaf.  Thereafter, the programmable controller waits for
access again from the computer, acknowledges access and
receives the information regarding the second cut.  This continues until the programmable controller receives information
regarding all n cuts to be made in leaf A.  At that time, the
programmable controller acknowledges that all information has
been received with respect to leaf A and proceeds to initiate
the controller for processing leaf A.  After leaf A has been
processed, the programmable controller then receives information regarding the cut positions and cutters from the digital
computer for making the cuts in the next leaf.  The cutting
operation is proceeding as the scanning operation for the
next leaf is proceeding.  FIGURES 4, 5 and 6 are to be taken
together to illustrate one arrangement for accomplishing the
system contemplated by the present invention.  The details
of the system will be hereinafter set forth.  However, it
should be appreciated that there are several machine control
arrangements and computer arrangements which can be employed
in practicing the method and system contemplated by the
description herein.

<div align="center">

SCANNING AND CREATING DIGITAL FACSIMILE DATA

(FIGURES 7, 7A, 7B and 8-12)

</div>

As explained with respect to the general description above,

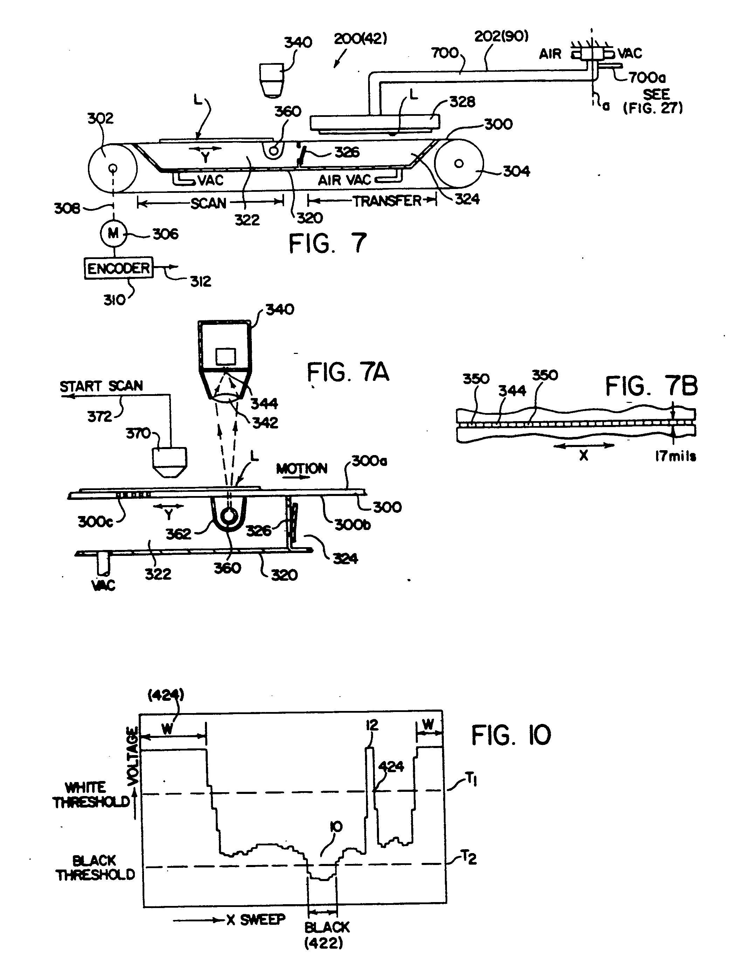

a system in accordance with the present invention involves an arrangement for obtaining digital information regarding the light transmitted characteristics at various identifiable locations or Pixels (X, Y locations) on a natural tobacco leaf L for processing of this information to select available cut positions for one or more cuts to be made in the tobacco leaf in the process of forming wrappers for cigars. As indicated in the block diagram of Figure 4, used for illustrating certain basic concepts of the system under consideration, block 42 contemplates a scanning mechanism schematically represented as mechanism 200 in the control concept block diagram shown in Figure 5. A variety of mechanisms 200 could be employed for the scanning operation to produce the desired digital information. One of the basic concepts of the illustrated system is the generation of a signal, preferably digital, indicative of the surface or color condition of selected, known locations or Pixels on the tobacco leaf, which Pixels can be combined and processed in a manner generally described above to give a representative facsimile, image or profile of the leaf including both color variation and defects such as holes and dark areas and stems. This facsimile, profile or image is then used to graphically or arithmetically locate the particular cut positions for subsequent cutting of wrappers from the natural leaf. To produce digital information or data regarding the surface condition or color variation at locations on leaf L, mechanism 200 is used. This mechanism is schematically illustrated in Figures 7, 7A, 7B, 8 and 9 which show the preferred scanning operation. In accordance with this illustrated embodiment, there is provided a continuously driven belt 300 movable around spaced support rolls 302, 304 and having an upper surface 300a and a lower or inner surface 300b. For the scanning operation, the belt is transparent or translucent so that it can transmit light rays through the belt for a purpose to be described later.
In practice, this belt is translucent and formed from
a material known as "revo" which is a clear nylon
belting supplied by L. H. Shingle Company and has a
general thickness of 0.04 inches (1 mm). This standard
nylon belting is provided with small perforations 300c
extending through the belting to develop a positive
pressure differential adjacent upper surface 300a when
a vacuum is created adjacent lower surface 300b. The
use of the vacuum at surface 300a holds leaf L in a
manually or machine spread condition on leaf receiving
surface or support surface 300a in the "scan" position
shown in Figure 7. To drive the belt in a clockwise
direction, as shown in Figure 7, there is provided an
appropriate electric motor 306 connected to roll 302 by
a drive mechanism schematically represented as dashed
line 308. An encoder 310 provides pulses in output
line 312 when motor 306 has driven belt 300 axially a
preselected distance. In practice, this preselected
distance is 0.05 inches (1.27 mm). Consequently, an
encoder pulse is provided after each movement of 0.05
inches of belt 300 in the Y direction indicated in
Figures 7 and 7A. Below the belt in both the "scan"
and "transfer" positions there is provided a vacuum box
320 of somewhat standard design in the cigar making
industry. This vacuum box substantially traverses the
"scan" and "transfer" positions of the upper run of belt
300 between rolls 302 and 304. If the leaf is to be
placed on belt 300 in the lower run, a vacuum box could
be provided along this run and roll 302 could be a
standard vacuum roll. Adjacent the scan position there
is provided a vacuum chamber 322 which is maintained at
a vacuum by an appropriate external vacuum source. In
the transfer position, there is provided a chamber 324
which combines with chamber 322 to form the total box 320. A pressure sensitive flapper valve 326 separates chambers 322, 324 to allow selective transfer of the leaf by a mechanism 202, which will be explained later. The leaf is to be removed from upper surface 300a of belt 300 in the transfer area after it has been scanned. Transfer chamber 324 can be provided with a vacuum or with pressurized air. After a leaf has been scanned, it is moved to the transfer position. At that position chamber 324 is pressurized. This allows the scanned leaf to be grasped by a vacuumized transfer head 328. This head has a lower vacuumized leaf receiving surface that holds the leaf L in an oriented position and transfers it to the cutting table in a manner to be described later. Thus, to transfer the leaf from belt 300 to transfer head 328 pressure is applied to chamber 324 and vacuum is created adjacent the lower surface of transfer head 328. This transfer head and procedure is used often in the tobacco industry to transfer tobacco products from a belt to another structure. When pressure is applied to chamber 324, valve 326 closes to prevent loss of vacuum in scanning chamber 322. Of course, other arrangements could be used for holding leaf L by vacuum onto a scanning surface while the surface is being moved and then for removing the leaf from the scanning surface; however, the arrangement illustrated in Figure 7 is now contemplated for use in the present system. Belt 300 is stopped momentarily for leaf transfer.

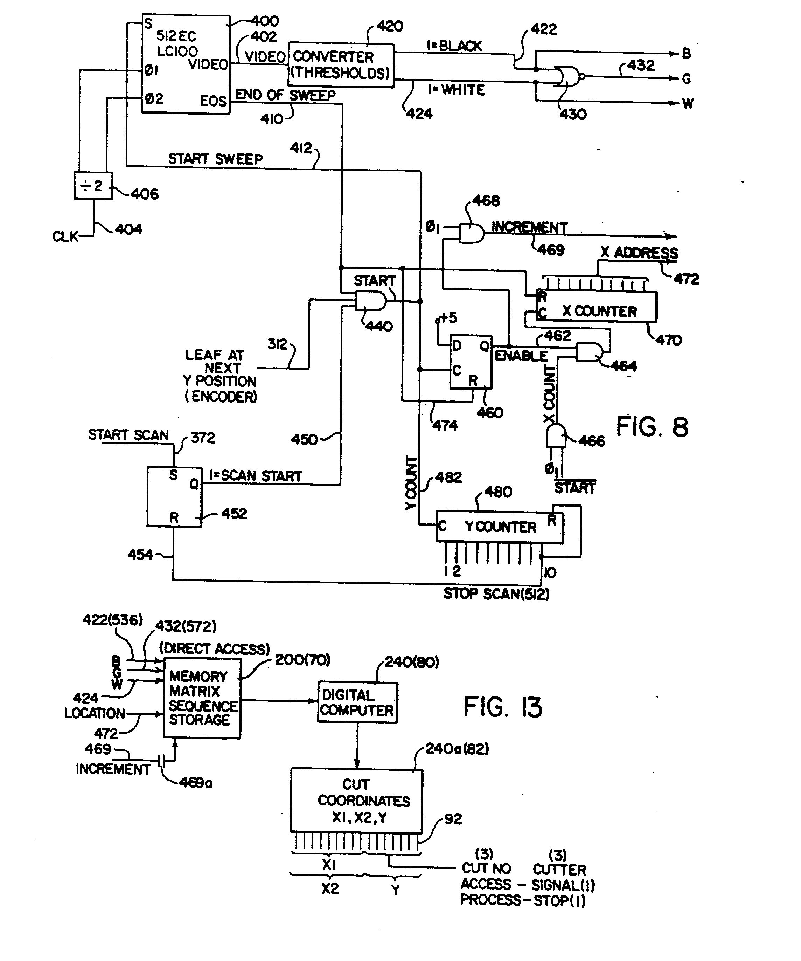

In accordance with the preferred embodiment of the present invention, scanning device 200 includes any appropriate mechanism for identifying the color content or the light intensity characteristics at various preselected locations or Pixels across the face of tobacco leaf L at X and Y positions. In practice, the data creating mechanism is a scanning head 340 which simultaneously records light intensity at spaced locations across belt 300 in a direction, identified as the X direction in Figure 7B.    The X direction is generally
orthogonal to the longitudinal or Y direction shown in
Figures 7 and 7A.    In essence, the scanning head 340
receives light rays along a selected Y position which
is indicative of the light intensity veiwed at given
spaced positions across the leaf.    The term "scanning"
as used in conjunction with mechanism 200 and head 340
means that these positions are read in series although
they may be simultaneously detected.    The scanning
feature is provided by the circuit shown in Figure 8.
The light intensity at most locations in both the X
and Y directions of leaf L is measured and an output
is created representative of this light intensity.    Of
course, certain approximations are acceptable.    Locations in the X direction are viewed by head 340 at
spaced lines or positions in the Y direction.    This
can create certain small bands of undetected areas
between adjacent Y positions.    This feature is mini-
mized by shifting or moving the scanned position only
a small distance in the Y direction.    This will provide
sufficient digital information or data for identifiable
locations on the surface of leaf L to create leaf
profile for location of the profiles 30.    In practice,
a LC 100 512 EC Reticon detecting or scanning head 340
is employed.    This type of unit is commercially available from Reticon Corporation of Sunnyvale, California.
In this type of mechanism, a lens 342 directs light
rays from the surface of leaf L into an elongated
aperture 344.    By using the lens, the view distance in
the X direction at the leaf area is focused onto 512
transversely spaced light intensity sensing units 350
each having a width approximately 2 mils (0.05mm).
Consequently, the light profile in the X direction at a
given Y position is focused on and changes the charged
characteristics of the light intensity sensitive units
350 schematically illustrated in Figure 7B.    In this

- 22 -  0031807

manner, light intensity at 512 different indexed or
shifted positions in the X direction of belt 300 are
read simultaneously by the separate units 350.   This
covers the total leaf width and much of surface 300a.
The Reticon unit uses a clocking pulse to step a
shift register which allows serial reading of the
light intensity data of each 512 units 350.   This
serial output is an analog video output as described
in Figure 8.   The voltage of this video output is an
analog representative of the light intensity exposed
to each of the units 350 spaced in the X direction at
a given Y position.   The setting of units 350 in a
given Y position is not scanning; however, the output
from the Reticon unit is a scanning function since it
reads successive cells or units 350 and outputs them in
series as representative of the light intensity
condition viewed by the units across a given Y position.
After all units 350 have been read, scanning unit 350
is ready to receive the light intensity at selected
positions across the next adjacent Y position of leaf L.
To do this, belt 300 moves leaf L by motor 306.   As
will be explained later, encoder 310 produces a signal
in line 312 which indicates that the units or cells 350
are now ready to read the light pattern across the next
successive longitudinally spaced position on surface
300a which carries and supports leaf L.   By progressing
the belt 300 in the Y direction, the output of the
Reticon sensing head 350 reads the identifiable transverse locations on surface 300a at the various orthogonally spaced positions in the Y direction.   The
spacing between the units 350 and leaf L is such that
a distance of approximately 12.5 inches (317.5mm)
is focused by lens 342 onto the 512 light sensitive
units.   This provides a spacing in the X direction of
approximately 0.025 inches (0.64mm) as compared to a
spacing of approximately 0.05 inches (1.27mm) in the Y
direction as determined by the adjustable setting of

0031807

encoder 312.    As is known, the 512 units or cells 350
are approximately 2 mils in width; therefore, the spacing
of the units from the leaf and the lens produces a scan
or viewing field in the X direction of slightly over
1:10.    Other arrangements could be used to change the
viewed field across leaf L.    The setting of unit 340
to provide a unit or cell 350 for each approximately
0.025 inches (0.64mm) has proven satisfactory and provides
an identifiable location, or a Pixel, which is satisfactory in size.    The Pixel has Y direction dimensions of
approximately 0.50 inches (1.27mm) to give good resolution for the ultimately constructed image or profile
of the leaf L.    The output of the Reticon unit for each
of the Pixels in the X direction is a video signal having
a voltage level indicative of the sensed light intensity
at the individual cells 350 being read by the Reticon
unit in accordance with standard practice for this type
of unit.    As so far described, scanning head 340 has
produced an analog signal for each of 512 positions
across the surface 300a of belt 300 for a given location
of the belt in a Y direction.    As the belt is moved by
motor 306 to a next position creating an encoder output
in line 312 this process of providing output analog
signals for 512 positions in the X direction is repeated.
This action is continued until the total leaf has been
processed.    In practice, there are 512 Y direction
positions to combine with the 512 X direction positions.
Since the Y direction positions are substantially twice
as great as the X direction positions, the identifiable
locations are more highly resolved in the X direction.
The surface area on belt 300 being viewed by scanning
head 340 measures approximately 12 inches (304mm) across
and about 25 inches (635 mm) in length.    This is sufficient to encompass natural tobacco leaves of the type
used in producing wrappers for cigars.

cigars. This monitored field of view of course can be changed by the optics of the scanning operation and by changing the number of Y direction positions used to complete a total scan of the surface 300a carrying a leaf L. The belt can move continuously at a rate allowing Pixel reading.

To provide an accurate light intensity profile of the total leaf, the preferred embodiment of the system uses a back lighting arrangement wherein light rays are passed simultaneously through belt 300 and leaf L. This procedure produces high resolution light intensity profile at the detecting cells 350. This back lighted profile is more nearly indicative of the actual condition of the surface of the leaf, together with certain conditions which may be within the leaf or on the opposite surface of the leaf. Thus, one aspect of the system, as contemplated for use in practice, is back lighting of the leaf to pass visible light rays or other detectable energy rays through both the belt 300 and leaf L. In accordance with this aspect of the novel system, there is provided a transversely extending apertured fluorescent light 360, with the elongated apertured window of the light extending generally parallel to the aperture 344 controlling light flow to the units or cells 350. This aperture in unit 340 is approximately 17 mils (0.43 mm) in width. The light _____

rays from the apertured fluorescent light 360 are
directed through belt 300 and leaf L, if the leaf is over
the light, and into aperture 344.   An apertured fluorescent light for use in the system is a stock item avail-
able from various sources and has an opening of approximately 30° and a reflective surface around the
remainder of the interior surface of the light.   This
gives a directed light source aligned with the aperture
344.   Light 360 is fixed with respect to scanning head
340 by an appropriate mounting arrangement including a
shield 362.   This shield further restricts the deflection of light from the area of the belt to be illuminated.
In Figure 7A, an appropriate device, such as a photocell 370, is used to detect the leading edge of leaf L
as it is conveyed by belt 300.   This photocell creates
a "start scan" signal in line 372 which will be used in
the circuit illustrated in Figure 8 for the purpose of
starting the scanning operation.

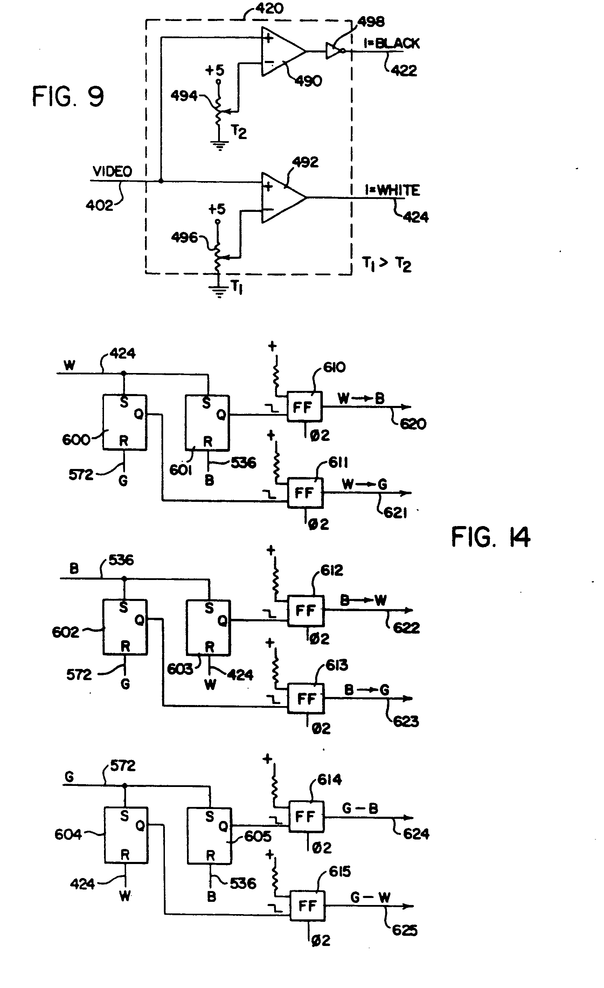

Referring now to Figure 8, a general scanning
and output logic diagram for obtaining digital output
facsimile data from the operation of scanner head 340
is illustrated.   In this illustrated logic circuit, the
standard Reticon reading device 400 is used to provide a
series of analog data bits on line 402 which correspond
to the X Pixels which are outputted in series by an
external clocking pulse on line 404.   These clocking
pulses are divided by an appropriate divider 406 to
produce two input clocks $\emptyset1$, $\emptyset2$ for controlling the
scanning operation of the sequential reading device 400.
This sequencing device employs a shift register to read
each of the different units or cells 350 and to output
an analog voltage on line 402 for each cell.   At the end
of a sweep across the cells, there is logic 1 produced
in the end of sweep (EOS) line 410.   The clock at line
404 may have a variety of frequencies; however, in
practice it is in the range of 100 Kilohertz to 1
Megahertz according to the speed of the output signal required. Since a subsequent sweep is controlled by movement of the belt 300, as shown in Figure 7, a rapid sweep through the 512 Pixels is employed. A conversion circuit 420, best shown in Figure 9, produces a digital logic 1 in line 422 when the light intensity of a Pixel being read produces an analog voltage in line 402 less than a preselected voltage level. This logic 1 is indicative of a "black" condition for a Pixel. In a like manner, if the analog voltage of a Pixel is above a certain voltage level, which is indicative of a white condition, such as no leaf or holes in the leaf, a logic 1 appears in line 424. This is termed a "white" Pixel condition. A white or black condition indicated by the logic in lines 422, 424 control NOR gate 430 which produces a "gray" digital signal in line 432 when there is neither a black or white signal. Thus, a Pixel being read produces either a black, white, or gray digital output signal indicative of the light intensity at the Pixel. The gray signal is generally indicative of the natural color of the leaf being exposed to a cell 350. At the end of each sweep, a logic 1 in line 410 stops the video output at line 402. The sequencer 400 is initiated for a subsequent sweep by a logic 1 at the start terminal S. To create a logic 1 in the start line 412, there is provided an AND gate 440 having three inputs. The first input 312 is the output of encoder 310 shown in Figure 7. A signal appears in line 312 when the leaf is in the next Y position for a subsequent scan in the X direction. The second input to gate 440 is line 410 which is enabled when a prior sweep has been completed. The third input is line 450 which is a logic 1 during the scanning operation. A logic 0 in line 450 prevents scanning. A variety of circuits could be used for controlling the logic of line 450; however, in the illustrated embodiment, a flip-flop 452 is set by a logic 1 in line 372 from the photocell 370 shown in Figure 7A. Thus, when a leaf is moved by belt 300 into the scanning position, a logic 1 appears in line 372. This sets flip-flop 452 to enable gate 440. After the desired number of positions in the Y direction have been processed, in practice 512, a logic 1 appears in line 454. This resets flip-flop 452 and shifts a logic 0 into the line 450. This disables gate 440. Sweep start signals in line 412 can not be created until the next leaf sets flip-flop 452. Consequently, at each Y position a logic 1 is created at the output of gate 440 by line 312, if a leaf is being scanned. This starts the next X sweep. A logic 1 in line 412 not only starts a sweep, but also clocks flip-flop 460 to produce an enable signal in line 462. This enables X counting gate 464, controlled by gate 466, and enables increment gate 468. This latter gate is optional for a purpose to be described later. A logic 1 in line 412 up counts Y counter 480 by pulsing Y count line 482. All of these functions are caused by a signal in line 312 as long as gate 440 is enabled by lines 410 and 450. As soon as a sweep has started, a logic 0 appears in the line 410 which inhibits gate 440 until the X sweep has been processed.

During an X sweep, output pulses are created in lines 422, 424 or 432. With each pulse, gate 466 clocks gate 464 to up count X counter 470. This produces the X address of the Pixel being read at the output of X counter 470. At the end of the X sweep, not only is gate 440 enabled, but X counter 470 is reset for the next sweep and flip-flop 460 is reset by line 474. This reset produces a logic 0 in the enable line 462 to disable counting gate 464 and incrementing gate 468. When Y counter 480 reaches a count of 512 the Y counter is reset. Line 454 resets flip-flop 452 to indicate that the scan is completed.    Flip-flop 452 then prevents
further scanning until the next leaf is available for
processing.

In operation, for each sweep, the light intensity value of the Pixel and the X address of the Pixel
is available at the output of the circuit shown in
Figure 8.    This information is available for each
sweep until the Y counter has indicated that the
desired number of X sweeps has been completed.    At
that time, the sweep circuit is deactivated until the
next leaf is available for processing.    The Y counter
could be used to input the Y addresses for any given
Pixel.    In other words, the X address in lines 472
from X counter 470 could be combined with the output of
Y counter 480 to give not only the X address for the
Pixel, but also the Y address.    The Y address is not
generally required in the illustrated embodiment of the
invention because it is sequenced in series.

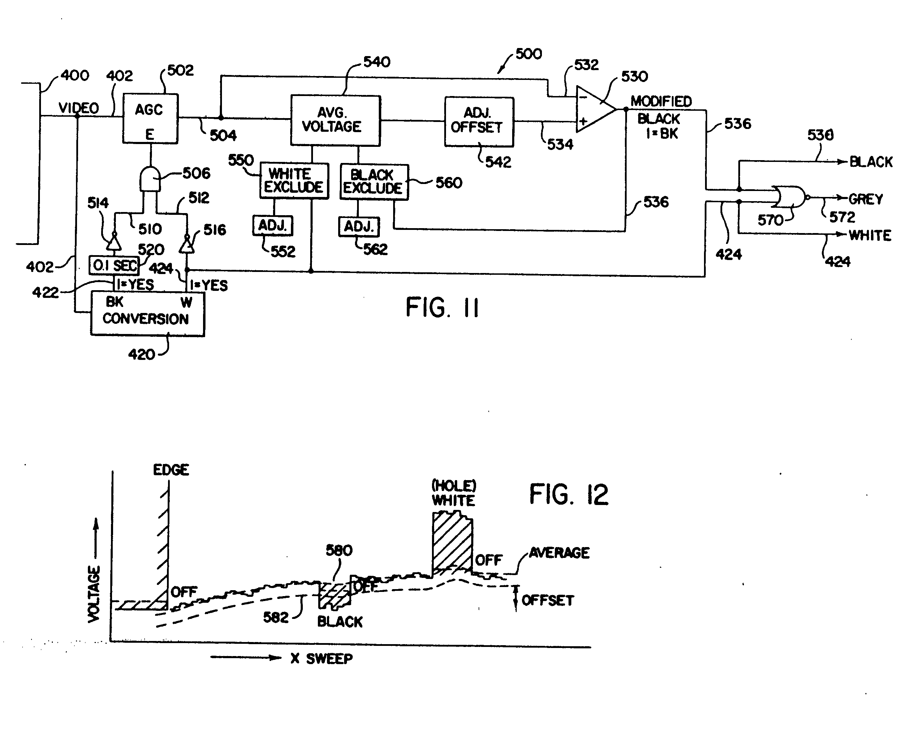

Referring now to Figure 9, the converter 420 is
schematically illustrated as including two differential
amplifiers 490, 492 poled as shown and having voltage
adjustable rheostats 494, 496 to control the respective
negative terminals.    An inverter 498 inverts the output
of amplifier 490 to produce a logic 1 in line 422 when
a condition designated as "black" by rheostat 494 has
been reached.    Other arrangements could be used to
convert the analog video signal in line 402 to a digital
logic indicative of the black or white conditions.
Referring now to Figure 10, this is a graph of a single
X sweep across the leaf at a selected Y position.    For
illustrative purposes, the leaf has a center stem 10 and
a hole 12.    It is noted that opposite edges of the leaf
are indicated by a white signal in line 424.    The black
stem is indicated by black signal in line 422.    The hole
12 is represented by a white signal in line 424.    Inbet-
ween these extreme positions, logic 1 appears in line 432.

This provides the general profile for the remainder of the leaf which does not exhibit either a drastically white or a drastically black condition. A sweep as illustrated in FIGURE 10 will be repeated 512 times for each leaf L and the information provided during each sweep can be used to create a profile of the leaf indicating the defect areas which should be avoided when locating a wrapper cut position on the leaf.

In practice, it was found that the procedure for obtaining black and gray Pixel information by comparing the light intensity with a fixed value for determining black presented some difficulties, especially when the color of the leaf changed or the conditions of the light or cells 350 varied. Consequently, in accordance with another aspect of this system a modified black signal is obtained by comparing the Pixel light intensity with a controlled average voltage. This average voltage uses the analog signals at line 402 for a majority of the Pixels. By comparing an analog Pixel voltage with an average voltage for prior Pixels, a black signal is recorded when there is in fact a substantial black condition compared with the operating conditions of the scanner and the color of the leaf. A circuit developed for obtaining this modified black signal is schematically illustrated in FIGURE 11. In this figure, the black modification conversion circuit 500 includes an automatic gain control, or amplifier, 502 which amplifies the analog Pixel voltage from line 402 and directs it to line 504. The automatic gain control or amplifier 502 is not needed in certain instances; therefore, it is enabled only when required by a gate 506. This gate has inputs 510, 512 connected by inverters 514, 516 with the output lines 422, 424, respectively, of conversion circuit 420 previously described. If the previously described conversion circuit produces a white signal, a logic 0 is directed to gate 506 and amplifier 502 is not activated. If a black signal is created in line 422 the automatic gain control or amplifier 502 is deactivated and held deactivated for a time delay indicated by the block 520. This time delay retains the black signal for a set time, which is 0.1 seconds in practice. In other words, gate 506 is deactivated whenever a white signal is created by circuit 420 and is held deactivated whenever a black signal is received. This allows the automatic gain control to be inactive when the sweep is mostly white, such as when there is no leaf or only a minor portion of the leaf is being detected by the sweep in the X direction. In this manner, the averaging concept does not become over weighted in a white voltage direction.

A differential amplifier 530 has inputs 532, 534 and an output 536 which receives the modified black signal. The voltage at input 534 is determined by the accumulated average of the Pixel voltage in line 504 as averaged by circuit 540. This is a capacitor averaging circuit in practice. The voltage of the capacitor averaging circuit is offset downwardly by an appropriate offset circuit 542. Consequently, the voltage at the input 534 is the average of the Pixel voltages, but offset downwardly for a reason to be explained later. To prevent the Pixel average voltage captured in circuit 540 from being distorted by white signals and black signals, there is provided a white exclusion circuit 550 which is adjustable as indicated by circuit 552. In a like manner, a black exclusion circuit 560 is provided with an adjustment circuit 562. Circuit 540 receives no signals when gate 506 is disabled because of mostly white being recorded. The circuit 550 removes those white areas which are not too large, such as a small hole.

The modified black output line 536 and line 424 are direct- ed to NOR gate 570 to produce a gray Pixel data in line 572 when there is no white or modified black signal. During normal X sweeps across the leaf, the amplifier 502 is operative. It is only inoperative when there is a prolonged period of white. This removes from the averaging function the predominantly white area of surface 300a surrounding the leaf being scanned or the white area of a large hole. As shown in FIGURE 12, averaging circuit 540 captures an average voltage indicated basically by the line 580. This average voltage line excludes the black portions and white portions indicated by the legend OFF either by disabling amplifier 502 or by exclusion circuits 550, 560. Offset circuit 542 offsets the average voltage downwardly to a level shown as line 582 which is the voltage used as a comparison voltage for the Pixel voltage in line 532 to determine a modified black output. By offsetting the average voltage downwardly, only light intensities which are lower than the offset average reference voltage in line 534 (line 582 of FIGURE 12) will produce a black signal. A white signal is produced in accordance with the conversion circuit 420 as previously described. By using the circuit as shown in FIGURE 11 and graphically depicted in FIGURE 12, a more accurate color profile in the X direction is obtained. As can be seen, the average voltage from a prior X sweep remains in the circuit 540 for the starting point of the next sweep. In this manner, if a group of dark leaves are being scanned, black defects can be detected. In such an instance, a fixed data voltage for detecting the black areas could indicate the total leaf is black. Of course, it would be possible to adjust the fixed black data for darker leaves or changes in the response of scanner 340 to overcome some of this difficulty; however, the circuit 500 of FIGURE 11 automatically makes appropriate compensations. FIGURES 9 and 11 could be modified to give more than one shade of gray if needed.

As so far explained, the scanning system creates three output signals which are black, gray and white that are digital signals with a given logic indicating the existence of one of these colors for a particular Pixel which has an X address for a given Y position.  As the scan is continued, the color profile of the total tobacco leaf being processed is obtained by a series of signals indicating the shielding effect or the light intensity of various identifiable locations or Pixels across the total surface of the leaf and also across those portions of surface 300a which are in the scanning pattern, but are not covered by a leaf.

GENERAL DATA PROCESSING

(FIGURES 4, 5, 6 AND 13)

As so far described, raw data is obtained for each Pixel or identifiable location during the scanning operation and this raw data is available at the output lines of the structure shown in Figure 8 or as modified by the showing in Figure 11.  This data includes the color intensity of a given location or Pixel and the address in the X direction across the surface 300a.  This information can be stored in sequence in standard READ/WRITE memory through a normal direct accessing to the memory units used in digital computers.  In other words, the raw data can be stacked in sequence in the storage unit 70, shown in Figure 4 which corresponds to the direct access memory 220 of the control concept as illustrated in Figure 5.  This feature of storing data relating to the color intensity of each Pixel is schematically illustrated in Figure 13 wherein the increment line 469 is used to index a direct accessed memory after each Pixel voltage signal is processed by the converter 420 and provided as a white, black or gray signal.  To allow time for settling of the Pixel information in lines 422 (536), 432 (572) and 424 a time delay arrangement can be provided.  This ————————————————————

is schematically illustrated as a time delaying capacitor
469a.  As so far described, Pixel data bits for all X Pixels
are stored in the direct access memory for each Y line or
position.  Since each of the Y lines is stored, the Y address
from counter 480 shown in FIGURE 8 is not required.  Indeed,
with the raw data being provided to the direct accessed
memory, the X address would not be needed since a particular
location in memory would be assigned to each of the Pixels
both in the X and Y directions.  As will be explained in
accordance with the illustrated embodiment of the disclosed
system, the X address is required because not all the Pixels
are stored.  In addition, the X address would be valuable
information for processing the leaf profile in most software
arrangements.  Digital computer 240 uses the stored profile
data in the memory unit to locate and arithmetically convert
cut coordinates as represented by block 240a in FIGURE 13.
This function block corresponds to the processing block 82
of FIGURE 4.  The cut coordinates employed in the preferred
embodiment are X1, X2 and Y which are used in the cutter
adjusting mechanism of the preferred embodiment as will be explained later.  In other words, each cut position is located
by three linear coordinates which are identified for the purposes of description as X1, X2 and Y.  These coordinates are
provided as eight bit digital words each of which will control
a linear moving mechanism in accordance with the magnitude of
the digital number contained in the word.  The digital computer also indicates the number of the cut and the particular
cutter used together with an ACCESS signal and a PROCESS stop
signal, as schematically illustrated in the general process
chart of FIGURE 6.  Essentially, the present system stores
data relating to the profile of the leaf and its defects
in a manner which can be processed to locate the cut positions within the created profile image of the leaf. The leaf itself is not used in the location of the cutter positions. The processing of stored data to locate the cut positions is well within the general technology of the computer art when the memory has been loaded in accordance with the present system. Hereinafter, certain programming techniques will be discussed which simplify the cut location procedure used by the digital computer; however, various other arrangements could be employed for the cut location to develop cut coordinates for each cut.

## TRANSITION CONVERSION
## (FIGURES 14 AND 14A)

As so far described, raw data indicative of the color of each Pixel has been created. This information could be used for controlling the cutting equipment as hereinafter described by locating the wrapper cut positions based upon this raw data. However, in accordance with the preferred embodiment of the invention, the raw data is not required. As long as there is raw data indicating white in the Pixels, the leaf has not been reached or a hole or white defect is being viewed. The same is true regarding a succession of gray or black raw data signals. Thus, to reduce the stored data necessary for constructing the profile or image of leaf L, the present system employs data indicative of color transitions. This transition data information can be obtained in a variety of ways between the output of surface shown in Figure 8 and the input to the storage memory. This involves a digital circuit interfacing the scanner with the memory unit. A digital circuit for creating transition data and usable between the scanning circuit of Figure 11 (Figure 8) and the memory units is illustrated in Figures 14 and 14A. Referring now to Figure 14, transition flip-flops 600-605 are reset when a pulse of the identified color is created.   These flip-flops
are set when a given color pulse is created.   Consequently, the resetting of one of the flip-flops to
create a logic O output indicates a color transition at
the Pixel being processed.   Upon the receipt of a
reset pulse, the transition storing flip-flops 610-615
are clocked to produce a transition signal in lines 620-
625 as indicated.   On each Ø2 pulse, the storage flip-
flops 610-615 are strobed back to the reset condition.
The input lines of the circuit shown in Figure 14 are
the output lines of the preferred embodiment circuit
shown in Figure 11.   The output lines 620-625 receive
a pulse when a transition is made from one color to
another color at a given Pixel during an X sweep across
surface 300a.   If the transition given both the "to"
and "from" colors is to be used in constructing the
leaf profile for processing, the signals in lines 620-625
could be used directly; however, in practice, it is only
necessary to know that there is a transition to a given
color such as black, gray or white.   Consequently,
lines 620-625 are assimilated to determine whether or
not there is a transition to black, to gray or to white.
Various intermediate digital circuits could be used for
this purpose; however, in Figure 14A OR gates 630-632
having outputs 633-635, respectively, are employed.
Gate 630 has an output when there is a transition to
black.   In a like manner, gate 631 has an output when
there is a transition to gray, and gate 632 has an output when there is a transition to white.   By using a
digital system for determining transitions before the
information is stored into the direct accessed memory,
a substantially reduced number of storage locations are
required to provide the necessary information for the
total profile of leaf L.   When transitions are being
used the X address appearing on line 472 of Figure 8 is
required.   This determines the X position at which a transition is made to a given color. If the stem is
being detected, there will be a transition to black
at a given X position and then a transition to gray
after the stem has been passed. The same arrangement is used for defects, surface variations and the
edge of the leaf to determine the profile of the leaf
without using the raw data of all Pixels. The computer would have access to the transition information;
therefore, the color of any Pixel between transitions
would be known from the stored transition information.
Consequently, transition data is used to save memory
capacity. The transition circuitry shown in Figures
14 and 14A is in a digital circuit between scanning
circuit 200 and direct access memory 202 as schematically shown in Figure 5.

### FACSIMILE DATA INTERFACE AND
### DIRECT ACCESS STORAGE THEREOF
#### (FIGURES 15-20)

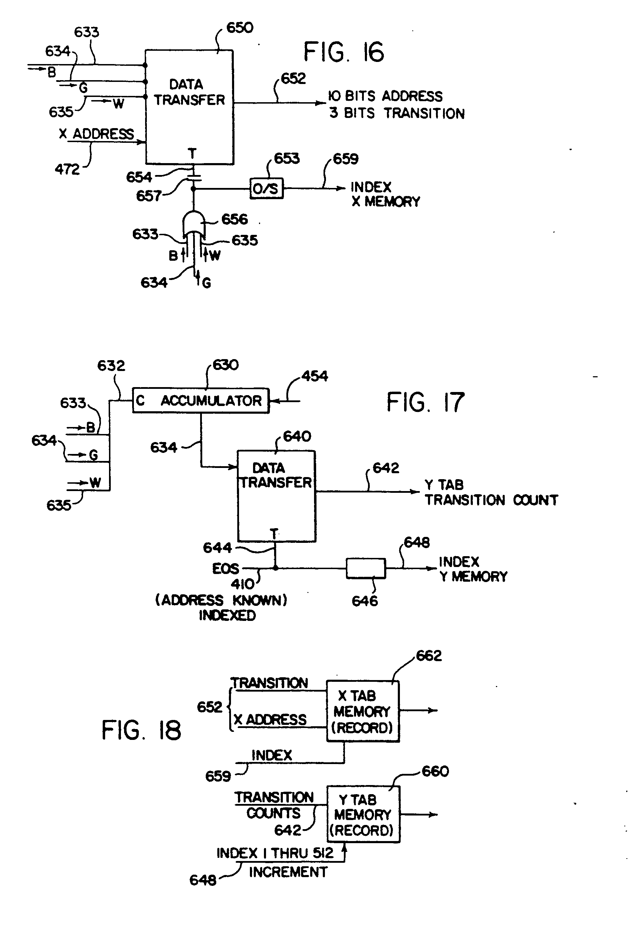

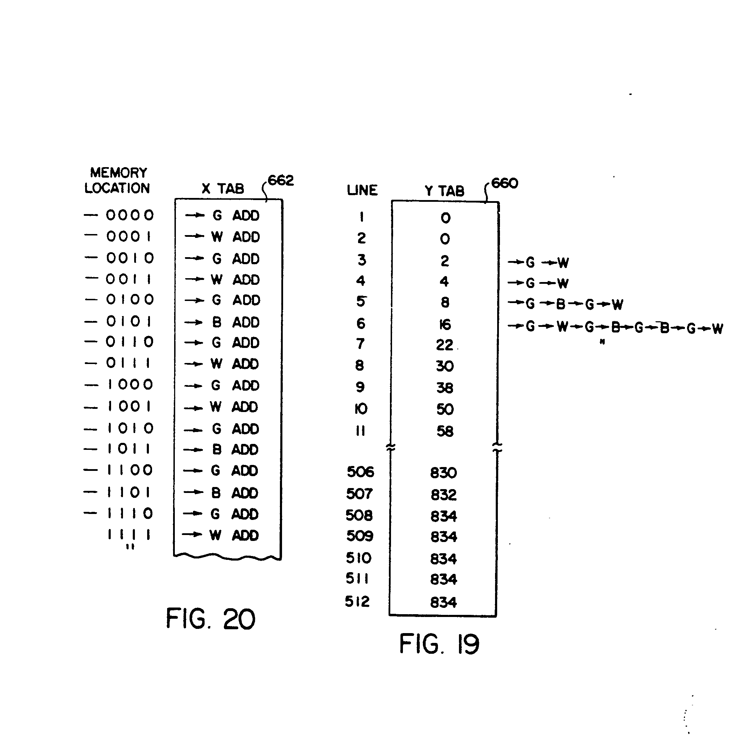

As indicated above, the raw data developed by
the scanning operation can be converted into transitions
by an intermediate digital circuitry between the output of the circuits of Figures 8 or 11 and the actual
memory used for storing the data. Also included in
this intermediate digital circuitry for processing
transitions instead of raw data developed by the scanning process are digital transition transferring devices
of the type schematically illustrated in Figures 15, 16
and 17. These transfer devices direct the transition
information or data to specific memory locations, as
schematically illustrated in Figrue 18. Figures 19
and 20 are schematic representations of the data actually stored in the memory. If the transition data is
indicative of the actual transition from one color to
another color, the data can be processed in accordance
with the digital circuitry illustrated in Figure 15.

In this circuitry, a sixteen bit accumulator 630 counts each transition pulse through a line 632. At the end of a scan, the reset line 454 resets accumulator 630. The output of the accumulator is illustrated as lines 634, which include sixteen lines to read the stages of accumulator 630. This digital data is directed to the input of a data transfer chip 640 having a sixteen bit output 642 and a data transfer terminal T activated by lines 644 which is the EOS line 410. To provide a certain time delay between the EOS pulse and the indexing of the direct accessed memory, there is provided a device, such as one shot device 646 having an output 648. The output indexes the memory to a next location into which the next accumulator data from lines 642 is stored. In operation, for each X sweep, the transitions are accumulated in accumulator 630. At the end of the sweep, the accumulated number of transitions is inserted into the memory and the memory is indexed. This continues for 512 sweeps in the X direction so that the memory has 512 sixteen bit words that are the accumulated number of transitions for each of the several Y positions of the scanning operation.

To transfer the transition data, the transitions and the X address thereof are directed to the inputs of a data transfer chip 650 having sixteen outputs 652 and a transfer terminal T controlled by line 654. OR gate 656 receives a pulse at each transition which pulse is delayed slightly by a device, such as capacitor 657, to then transfer the input data to the output lines 652 of transfer chip or device 650. An appropriate time delay 658 then allows indexing of the X memory unit by a signal in line 659. Thus, at each transition in the X direction, the address of the transition appearing in line 472 is transferred through the sixteen bit output 652 together with the type of transition. Thus, the X memory unit receives a transition and the Pixel address of the transition.

The circuitry illustrated in Figures 16 and 17 is the same circuitry and has basically the same numbers as the circuitry illustrated in Figure 15. The difference is that the transition information is received from the network illustrated in Figure 14A. There is only three bits of transition information or data, which data determine and record the type of transition. Accumulator 630 of Figure 17 still accumulates the same number of transitions; however, the data being transferred through the sixteen bit lines 652 is indicative of the transition to a particular color and not necessarily from which color the transition is being made. The circuitry shown in Figures 14, 14A, 16 and 17 is the preferred embodiment. This circuitry is interposed between the raw data scanning circuits of Figures 8 and 11 and the direct accessing memory units. This is schematically illustrated in Figure 18 wherein the direct access memory unit for the Y information is called the "Y TAB" memory 660. The accumulated number of transitions at each of the Y positions is recorded and stored in sequence in this unit. In a like manner, the X information including the type of transition and the Pixel address for the transition is directed to the "X TAB" memory 662. This memory is indexed at each transition. Thus, the Y tab includes the accumulated number of transitions for each of the 512 different Y lines being scanned by a scanner mechanism 200. The X TAB memory location includes the Pixel at which a transition is made by an X address and the type of transition. By using both the information and the X tab and Y tab direct access memory units, a profile of the leaf is stored for processing by an appropriate software arrangement for locating profile 30 within locations of the leaf L where there are no defects. The general outline of the memory data in the Y tab and X tab storage units or memories are schematically set forth in FIGURES 19, 20, respectively.

## PREFERRED DATA PROCESSING CONCEPTS
## (FIGURES 21-26)

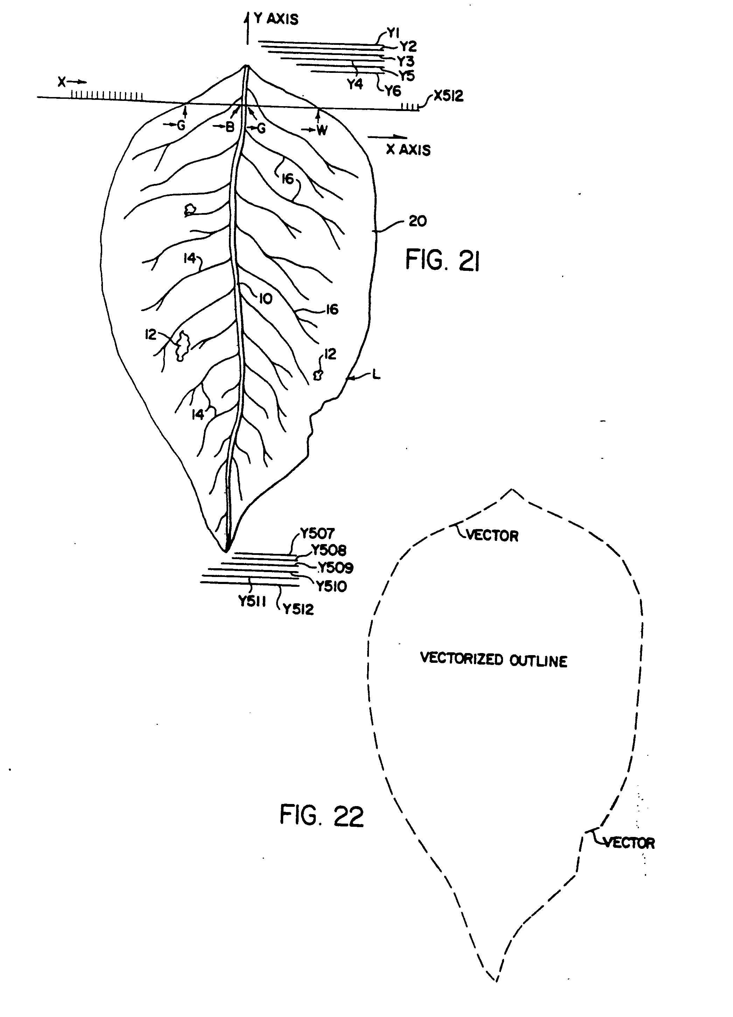

As previously explained, the raw data or transition data, in the preferred system, is transferred to the memory 200 for use by the digital computer 240. The units 660-662 of the preferred example correspond to memory 200 of the control diagram of FIGURE 5. The computer then creates the cut locations and computes the cut coordinates for the various wrapper cuts to be made from leaf L. This can be done by a variety of software packages; however, in accordance with the present system certain concepts have been developed for use in reducing the computer time and the software complexity. These basic concepts are set forth in FIGURES 21-26. Referring now to FIGURE 21, leaf L is shown with the X and Y coordinates of the scanning grid. In the single illustrated line X, it is noted that there is a transition to gray at the leaf edge, a transition to black at stem 10, a transition to gray after the stem and then a transition to white at the opposite leaf edge. This type of pattern continues through the total X sweep. Other transitions are also experienced. After the leaf has been passed, the subsequent X sweeps do not provide further transitions. At the first X sweep, there is usually no transition. Thus, the Y tab storage as shown in FIGURE 19 can indicate the length of the leaf. Also, the Y tab can locate the X transitions for a given Y scan by pointing to a required X tab location. For instance, if the sixth Y line is being read, the Y tab indicates that the X sweep is between the 8th and 16th transition of the X tab. The X tab is scanned between the 8th transition and the 16th transition to give the X sweep for the sixth Y line.
If the 11th Y line is to be analyzed, the X tab
between the 50th and 58th transition provides the X
sweep profile. This concept provides an easy access
to the X sweep profile for processing by the computer
by using transitions and accumulated transitions for
locating information in the X tab. There is no need
for a Y tab address although it is available, since
the Y tabs are serially stored data between 0-512.
By taking this information, in accordance with the
preferred data processing system, a vector of the
leaf outline shown in Figure 22 is created by
analytical geometry. This outlining vector concept
provides two X coordinates at a given Y line. The
first X coordinate is a first transition from white
and the second X coordinate is a last transition to
white. Thereafter, the Y line is indexed a set
number, such as 20-40 lines. The next Y line gives
two additional outline X coordinates. These X
coordinates are then noted and a mathematical vector
is created in the computer for comparing cut profile
30 (in vector form) with the vectorized leaf outline
to prevent interference with the leaf edge.

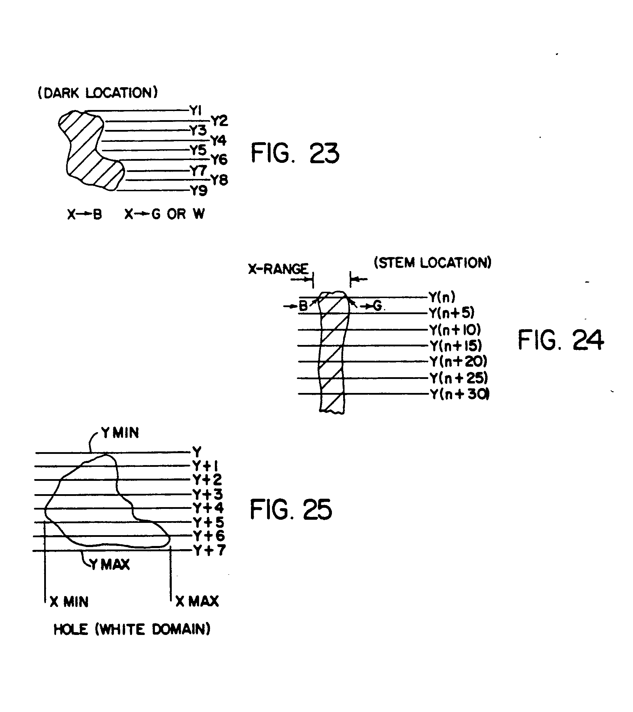

A dark area is located and recorded as shown
in Figure 23. Each of the Y lines is scanned for a
transition to black and then for a transition to gray
or white. . This indicates a black area on the leaf at
each of the Y lines. To locate holes in the leaf,
each of the Y lines is scanned for a transition to
white and then to gray or black. For each hole, a
maximum and minimum X and a maximum and minimum Y is
recorded in the memory profile to outline a hole area
or domain which is rectangular and is to be avoided
by a cut. A stem is located by noting a series of
transitions to black and then to gray along the stem.
If these transitions are aligned in the X direction
for a successive number of Y positions, _____

such as 5-10 lines, it is noted that the stem has been located for storing in a memory unit of the computer. If the transitions do not align in the X direction over successive Y lines, the transitions are not the stem 10 and are known to be either the diagonal veins or dark locations previously recorded. By vectorizing the outline of the leaf, constructing a rectangle or domain around the holes, noting the black or dark locations and locating the stem, there is sufficient processed information to locate the coordinates of a cut for a wrapper to avoid the stem, dark areas, white areas and holes, as well as the outline of the leaf. Figures 22-25 disclose concepts used to facilitate the creation of a total leaf facsimile in X and Y coordinates and vectors therebetween for location of proper cut positions. These are novel processing concepts and are not mathematical in nature.

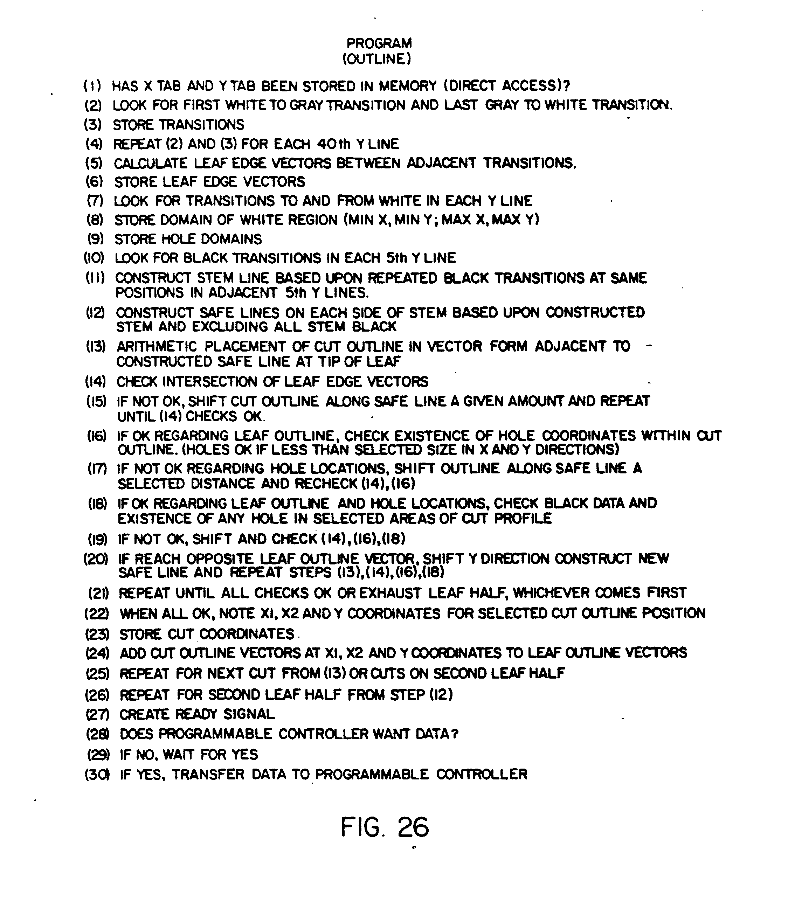

In Figure 26, the general programming approach to the preferred embodiment of the invention is illustrated. Basically these program steps which could be set forth in block diagrams are self-explanatory in nature when considering Figures 19-25 and can be accomplished by various software routines and techniques. The concepts of Figures 19-25 are novel procedures which can be performed by software and used in the general program of Figure 26.

In the general program outline, after the X and Y tab have been stored in memory, steps (2)-(6) create the vector outline of the leaf as shown in Figure 22. Steps (7)-(9) provide the hole domain as shown in Figure 25. Steps (10) and (11) construct the stem for the leaf which is to be avoided in the cut position. By an optimizing subroutine, a safe line is constructed adjacent both sides of the stem as shown in step (12) which safe line is to be avoided in positioning a cut profile 30. In step (13), the vectorized profile 30 is mathematically positioned at one end of the leaf and adjacent the stem safe line. In step (15), if there is an intersection of the edge vector as tested in step (14), the cut profile is shifted along the safe line a given amount and step (14) is repeated. If the outline is avoided as indicated in step (16), the existence of holes or other unwanted defects is determined. If there are holes or other defects not wanted in the profile 30, it is then shifted in the Y direction along the safe line adjacent the stem as in step (17) until a position is located wherein there is no hole location or leaf outline intersection. At that time, as indicated in step (18), the black area and any minute holes in the tuck or head are tested as indicated in step (18). As indicated in step (19), if the tuck or head has black areas or other small defects the shifting process continues until the cut profile vectors reach the opposite leaf vector outline or a cut location has been determined. If the outline has been reached without a cut location, a new safe line is constructed as indicated in step (20) which is offset (in the X direction) and spaced from the previous safe line. The process is repeated until whole leaf half has been exhausted as indicated in step (21) or a cut location has been obtained. If the cut location has been obtained and meets the parameters previously discussed, the X and Y position of a point on the profile 30 and the angle of this line with respect to the Y axis is recorded and the necessary X1, X2 and Y coordinates are calculated and stored for subsequent outputting to the programmable controller to use for subsequent cutting. The angle $\emptyset$ occurs because the stem may not be aligned in a Y line which will give an angled safe line. This angle is small since the leaf L is aligned as best shown in Figure 21 with the stem as vertical as practical. The type of coordinates which are required are correlated with the type of movement mechanism being
used for the cutting device so that the mathematics for
creating coordinates will create an output that
controls the position of the cutting in accordance with
the selected cut position originally identified as X,
Y and $\emptyset$. In other words, the X1, X2 and Y coordinates
are calculated from the X, Y and $\emptyset$ coordinates to corr-
espond with the exact type of cutting mechanism used
to adjust the leaf supporting surface or cutting surface with respect to the cutter as will be explained
later. This mathematical relationship is easily programmed into the computer. This function is steps (22)
and (23). After a cut position has been located, as
indicated in step (24), the vectors used in the profile
30 are added to the outline vectors of the leaf to
change the profile of the leaf vectors by excluding
from future consideration the previously selected cutting position. Thereafter, as indicated by step (25),
the process from step (13) is repeated to locate a next
cut or cuts on the half of the leaf being so far processed. After all the cuts have been located on one
half of the leaf, the steps commencing at step (12) are
repeated for the second half of the leaf. Thereafter,
the program has been completed and the computer will
create a ready or access signal as indicated by step
(27). This can be an output flip-flop or other
arrangement to indicate to the programmable controller
that the cut coordinates have been determined for a
particular leaf being scanned and processed. As indicated in steps (28)-(30), the computer then waits until
the programmable controller 210 indicates that it is
ready to receive the cut coordinates, the cutter and
cut numbers from the digital computer for use in subsequently cutting cigar wrappers from leaf L. Of course,
other programming concepts could be used in practicing
the present system, but the information illustrated in Figures 18-26 is preferred and involves certain novel concepts which reduce the software involved, decrease the cycle time for the total software program and reduce the chances of any error in the location of the cut positions for various cigar wrappers from a given natural tobacco leaf L. Basically, the outputted coordinates X1, X2 and Y are binary words that are transferred from the digital computer to the programmable controller for controlling the cut location determined by the computer. The digital information from the computer is then used directly for the location of the cut positions in a manner described in Application No. 79300323.7.

CLAIMS:

1. An apparatus for detecting the general outline and abrupt surface variations of a flaccid sheet-like workpiece formed from at least a portion of a natural tobacco leaf (L), said apparatus characterised in that it includes a foraminate, energy ray conducting workpiece receiving element (300) having a first workpiece supporting surface (300a) with an area and shape capable of receiving said workpiece in a spread condition and a coterminous second surface (300b); means (320) for creating a positive pressure differential from said first surface to said second surface in order to hold a workpiece against said first surface; an energy ray source means (360) on one side of said element (300) for passing energy rays through said workpiece receiving element (300) and through said workpiece (L), such that the transmitted energy intensity at the other side of said element is indicative of the shielding effect of said receiving element and said workpiece; a series of energy intensity measuring units (350) extending in a first direction (X) across said receiving element (300) and spaced from said first surface (300a) for measuring the transmitted energy intensity; means for creating (420, 430) a digital signal indicative of the shielded energy intensity at each of said units; means (306) for shifting the relative position of said units and said leaf in a direction generally orthogonal to said first direction; means (200; 660, 662) for recording said digital signals or further digital signals derived therefrom at each relative position of said units and said leaf; and means (240) for creating a representation of said general outline and abrupt surface variations based upon said signals.

2.      An apparatus as claimed in claim 1, characterised in that said shifting means includes means (306) for shifting said receiving element (300) with respect to said units (350).

3.      An apparatus as claimed in claim 1 or 2 characterised in that said energy ray source is an elongate light (360) and including means (362) for fixedly mounting said light (360) adjacent said second surface (300b) and aligned with said units (350).

4.      An apparatus as claimed in claim 1, 2 or 3, characterised in that said means for creating said digital indicative signals includes means (400) for creating said signals in series.

5.   -   An apparatus as claimed in claim 4, characterised by sensing means (410) for indicating when said series of signals has been created and means (412) responsive to said sensing means for activating said shifting means.

6.      An apparatus as claimed in claim 1, 2 or 3, characterised in that said means for creating said digital indicative signals includes scanning means (340) for creating a scanned series of digital signals indicative of the shielded energy intensity at each of said units; means (400) for cycling said scanning means through a cycle; scan starting means (440) responsive to said shifting means (306) for activating said cycling means through a successive scanning cycle.

7.      An apparatus as claimed in claim 6, characterised by means (410) responsive to the end of each scanning cycle for allowing operation of said scan starting means (440).

8. An apparatus as claimed in claim 6 or 7, characterised by a digital counter means (464) for counting each of said digital signals, a decoder means (470) for creating a decoded digital signal (472) indicative of each of said signals and means for using said decoded signal and said digital signal for creating said representation.

9. An apparatus as claimed in claim 8, characterised by a second digital counter means (482) for counting each of said shifts in said relative position and a second decoder means (480) for creating a second digital signal indicative of each of said shifts and means for using said second decoded signal and said digital signals after each said shift for creating said representation.

10. An apparatus as claimed in any one of claims 6 to 9, wherein said digital signal scanning means includes means (420, 430) for creating a first analyzed digital signal (424) when the energy intensity of a measuring unit (350) reads a value exceeding a preselected level, a second analyzed digital signal (422) when the energy intensity of a measuring unit reads a value less than a preselected value and means for creating a third analyzed digital signal (432) when the light intensity of a measuring unit is between said preselected values.

11. An apparatus for detecting the general outline and abrupt surface variations of a flaccid sheet-like workpiece formed from at least a portion of a natural leaf (L), said apparatus characterised in that it includes: a foraminate, visible light ray conducting workpiece receiving element (300) having a first workpiece supporting surface (300a) with an area and shape capable of receiving said workpiece in a spread condition and a coterminous second surface (300b); means (320) for creating a positive pressure differential from said first surface to said second surface whereby a workpiece is held against said first surface; a light source means (360) on one side of said element for passing light rays through said workpiece receiving element (300) and through said workpiece (L) such that the transmitted light intensity at the other side of said element is indicative of the outline and surface conditions of said workpiece; a light sensitive transducer means (340) for receiving said light rays on said other side of said element (300), said transducer means including a plurality of light responsive transducers (350) juxtapositioned along a given path and means (362) for directing light rays from only a finite linear portion of said workpiece receiving element (300) to said given path; means (306) for incrementally changing said finite linear portion sequentially from position-to-position along said workpiece; means (400) for reading the conditions of said transducers at each position of said finite linear portion; means (492) for sensing when one of said conditions of said transducers exceeds a first light intensity condition; and, means (490) for sensing when one of said conditions of said transducers is less than a second light intensity condition.

FIG. 1

FIG. 2

FIG. 3

FIG. 4

SPREAD LEAF ON VACUUM SUPPORT SURFACE — 40

LINEAR INDEXING INCREMENTED SCAN OF LIGHT PASSING THROUGH LEAF AT INDENTIFIABLE LOCATIONS — 42

INTENSITY AT LOCATION — 44

CONVERT LOCATION ANALOG DATA TO DIGITAL DATA — 50

SEQUENCER — 60

DIGITAL DATA — 72

SEQUENCE STORAGE UNIT FOR DIGITAL SCAN DATA — 70

LOCATION DATA — 74

LOCATION DATA — 46

INCREMENT — 469

LOCATE CUTS X Y Ø — 80

TRANSFER LEAF TO CUT TABLE — 90

LOCATE CUTTER WITH TABLE FOR CUT #1 — 102 | DIGITAL TO ANALOG MOVEMENT — 100

GENERATE COORDINATES OF CUTS X1, X2, Y — 82

92

CUT WRAPPER — 104

REMOVE CUT WRAPPER FROM TABLE — 106

STORE CUT WRAPPER — 108

CUT #2···N — 110

FIG. 5

PROGRAM CONTROLLER — 210

SCAN MECH. — 200

LEAF TRANSFER MECH. — 202

LOCATE CUT MECH. — 204

REMOVE WRAPPER MECH. — 206

REMOVE CUT LEAF — 208

HEWLETT PACKARD HP-2112

DIGITAL COMPUTER — 240

DIRECT ACC. MEM. — 220

92

2/13

0031807

INTERFACE — 92

Ⓐ

LEAF A
ACKNOWLEDGE ACCESS

STORE CUT NO. I

STORE CUTTER

STORE XI-I

STORE X2-I

STORE Y-I

WAIT FOR ACCESS

ACKNOWLEDGE ACCESS

STORE CUT NO. 2

STORE CUTTER

STORE XI-2

STORE X2-2

STORE Y-2

(N-CUTS)

ACKNOWLEDGE STOP

INITIATE MACHINE
CONTROLLER
LEAF A

FIG. 6

0031807

4 / 13

FIG. 7

FIG. 7A

FIG. 7B

FIG. 10

0031807

5 / 13

FIG. 8

FIG. 13

0031807

6 / 13

FIG. 9

420

498

+

−

490

494

+5

T₂

I=BLACK

422

VIDEO

402

492

+

−

I=WHITE

424

496

+5

T₁

T₁ > T₂

W

424

S Q

600

R

572 G

S Q

601

R

536 B

+

FF 610

Ø2

W → B

620

+

FF 611

Ø2

W → G

621

FIG. 14

B

536

S Q

602

R

572 G

S Q

603

R

424 W

+

FF 612

Ø2

B → W

622

+

FF 613

Ø2

B → G

623

G

572

S Q

604

R

424 W

S Q

605

R

536 B

+

FF 614

Ø2

G − B

624

+

FF 615

Ø2

G − W

625

FIG. II

FIG. 12

# FIG. 15

632  630  454  (OPTIONAL)

C  ACCUMULATOR  R

620  W→B
621  W→G
622  B→W
623  B→G
624  G→B
625  G→W

634

(16 LINES)

640

DATA
TRANSFER

T

642  Y TAB
DATA (16)
CHANNEL A

410  644
EOS

646  O/S

648  INDEX
Y MEMORY

X ADDRESS (10)  472

W→B  620
W→G  621
B→W  622
B→G  623
G→B  624
G→W  625

650

DATA
TRANSFER

T

652  X TAB
DATA (16)
CHANNEL B

656

654

657

658  O/S

659  INDEX
X MEMORY

# FIG. 14A

620  W→B
624  G→B

630  B  →B  633

621  W→G
623  B→G

631  G  →G  634

622  B→W
625  G→W

632  W  →W  635

0031807

9 / 13

FIG. 16

FIG. 17

FIG. 18

| MEMORY LOCATION | X TAB 662 |
|---|---|
| — 0 0 0 0 | → G ADD |
| — 0 0 0 1 | → W ADD |
| — 0 0 1 0 | → G ADD |
| — 0 0 1 1 | → W ADD |
| — 0 1 0 0 | → G ADD |
| — 0 1 0 1 | → B ADD |
| — 0 1 1 0 | → G ADD |
| — 0 1 1 1 | → W ADD |
| — 1 0 0 0 | → G ADD |
| — 1 0 0 1 | → W ADD |
| — 1 0 1 0 | → G ADD |
| — 1 0 1 1 | → B ADD |
| — 1 1 0 0 | → G ADD |
| — 1 1 0 1 | → B ADD |
| — 1 1 1 0 | → G ADD |
| 1 1 1 1 | → W ADD |

FIG. 20

| LINE | Y TAB 660 | |
|---|---|---|
| 1 | 0 | |
| 2 | 0 | |
| 3 | 2 | →G →W |
| 4 | 4 | →G →W |
| 5 | 8 | →G →B →G →W |
| 6 | 16 | →G →W →G →B →G →B →G →W |
| 7 | 22 | |
| 8 | 30 | |
| 9 | 38 | |
| 10 | 50 | |
| 11 | 58 | |
| 506 | 830 | |
| 507 | 832 | |
| 508 | 834 | |
| 509 | 834 | |
| 510 | 834 | |
| 511 | 834 | |
| 512 | 834 | |

FIG. 19

Y AXIS

Y1
Y2
Y3
Y4 Y5
Y6
X512

X →

G → B ← G → W

X AXIS

16

14

20

FIG. 21

10

16

12

12

L

14

Y507
Y508
Y509
Y510
Y511 Y512

VECTOR

VECTORIZED OUTLINE

FIG. 22

VECTOR

(DARK LOCATION)

Y1
Y2
Y3
Y4
Y5
Y6
Y7
Y8
Y9

FIG. 23

X→B    X→G OR W

X-RANGE          (STEM LOCATION)

→B          →G        Y(n)
                      Y(n+5)
                      Y(n+10)
                      Y(n+15)     FIG. 24
                      Y(n+20)
                      Y(n+25)
                      Y(n+30)

Y MIN

Y
Y+1
Y+2
Y+3
Y+4        FIG. 25
Y+5
Y+6
Y+7

Y MAX

X MIN          X MAX

HOLE (WHITE DOMAIN)

## PROGRAM
## (OUTLINE)

(1) HAS X TAB AND Y TAB BEEN STORED IN MEMORY (DIRECT ACCESS)?

(2) LOOK FOR FIRST WHITE TO GRAY TRANSITION AND LAST GRAY TO WHITE TRANSITION.

(3) STORE TRANSITIONS

(4) REPEAT (2) AND (3) FOR EACH 40th Y LINE

(5) CALCULATE LEAF EDGE VECTORS BETWEEN ADJACENT TRANSITIONS.

(6) STORE LEAF EDGE VECTORS

(7) LOOK FOR TRANSITIONS TO AND FROM WHITE IN EACH Y LINE

(8) STORE DOMAIN OF WHITE REGION (MIN X, MIN Y; MAX X, MAX Y)

(9) STORE HOLE DOMAINS

(10) LOOK FOR BLACK TRANSITIONS IN EACH 5th Y LINE

(11) CONSTRUCT STEM LINE BASED UPON REPEATED BLACK TRANSITIONS AT SAME POSITIONS IN ADJACENT 5th Y LINES.

(12) CONSTRUCT SAFE LINES ON EACH SIDE OF STEM BASED UPON CONSTRUCTED STEM AND EXCLUDING ALL STEM BLACK

(13) ARITHMETIC PLACEMENT OF CUT OUTLINE IN VECTOR FORM ADJACENT TO CONSTRUCTED SAFE LINE AT TIP OF LEAF

(14) CHECK INTERSECTION OF LEAF EDGE VECTORS

(15) IF NOT OK, SHIFT CUT OUTLINE ALONG SAFE LINE A GIVEN AMOUNT AND REPEAT UNTIL (14) CHECKS OK.

(16) IF OK REGARDING LEAF OUTLINE, CHECK EXISTENCE OF HOLE COORDINATES WITHIN CUT OUTLINE. (HOLES OK IF LESS THAN SELECTED SIZE IN X AND Y DIRECTIONS)

(17) IF NOT OK REGARDING HOLE LOCATIONS, SHIFT OUTLINE ALONG SAFE LINE A SELECTED DISTANCE AND RECHECK (14),(16)

(18) IF OK REGARDING LEAF OUTLINE AND HOLE LOCATIONS, CHECK BLACK DATA AND EXISTENCE OF ANY HOLE IN SELECTED AREAS OF CUT PROFILE

(19) IF NOT OK, SHIFT AND CHECK (14),(16),(18)

(20) IF REACH OPPOSITE LEAF OUTLINE VECTOR, SHIFT Y DIRECTION CONSTRUCT NEW SAFE LINE AND REPEAT STEPS (13),(14),(16),(18)

(21) REPEAT UNTIL ALL CHECKS OK OR EXHAUST LEAF HALF, WHICHEVER COMES FIRST

(22) WHEN ALL OK, NOTE X1, X2 AND Y COORDINATES FOR SELECTED CUT OUTLINE POSITION

(23) STORE CUT COORDINATES

(24) ADD CUT OUTLINE VECTORS AT X1, X2 AND Y COORDINATES TO LEAF OUTLINE VECTORS

(25) REPEAT FOR NEXT CUT FROM (13) OR CUTS ON SECOND LEAF HALF

(26) REPEAT FOR SECOND LEAF HALF FROM STEP (12)

(27) CREATE READY SIGNAL

(28) DOES PROGRAMMABLE CONTROLLER WANT DATA?

(29) IF NO, WAIT FOR YES

(30) IF YES, TRANSFER DATA TO PROGRAMMABLE CONTROLLER

## FIG. 26